# EUROPEAN PATENT APPLICATION

(11) **EP 1 661 577 A1**
(43) Date of publication of application: **31.05.2006**
(21) Application number: 04771912.5
(22) Date of filing: 13.08.2004
(51) Int. Cl.: A61K 38/00, A01K 67/027, C12N 5/10, C12N 15/09

(54) **THERAPEUTIC PREPARATION FOR HEMATOPOIETIC DISEASE**

(30) Priority: 13.08.2003 US 495001 P
(71) Applicant: Locomogene, Inc., Tokyo 105-0001 (JP)
(72) Inventor: NAKAJIMA, Toshihiro, Kohoku Garden Hills A-503, Yokohama-shi, Kanagawa 2240001 (JP); AMANO, Tetsuya, Tama-ku, Kawasaki-shi, Kanagawa 2140005 (JP); YAGISHITA, Naoko, Yokohama-shi, Kanagawa 2340053 (JP)
(86) International application number: PCT/JP2004/011951
(87) International publication number: WO 2005/016367

(57) **Abstract**

The present invention provides therapeutic formulations for hematopoietic diseases comprising as effective ingredient, synoviolin which is reported to be isolated as a synovial cell protein and its gene, and therapeutic methods and such comprising the step of administering the protein or polynucleotide. The present invention also provides as models of hematopoietic diseases, animals whose synoviolin is homozygously deficient and cells derived from such animals; and additionally provides methods that use these models in the screening of therapeutic agents for hematopoietic diseases.

SEQUENCE LISTING

<110> LOCOMOGENE, INC.

<120> THERAPEUTIC PREPARATION FOR HEMATOPOIETIC DISEASE

<130> BHP-A0301P

<140>
<141>

<150> US 60/495,001
<151> 2003-08-13

<160> 4

<170> PatentIn Ver. 2.0

<210> 1
<211> 3374
<212> DNA
<213> Homo sapiens

<220>
<221> CDS
<222> (403)..(2256)

<400> 1
gccctttctt atgagcatgc ctgtgttggg ttgacagtga gggtaataat gacttgttgg 60
ttgattgtag atatagggct ctcccttgca aggtaattag gctccttaaa ttacctgtaa 120
gattttcttg ccacagcatc cattctggtt aggctggtga tcttctgagt agtgatagat 180
tggttggtgg tgaggtttac aggtgttccc ttctcttact cctggtgttg gctacaatca 240
ggtggcgtct agagcagcat gggacaggtg ggtaagggga gtcttctcat tatgcagaag 300
tgatcaactt aaatctctgt cagatctacc tttatgtagc ccggcagtcg cgcggattga 360
gcgggctcgc ggcgctgggt tcctggtctc cgggccaggg ca atg ttc cgc acg 414
gca gtg atg atg gcg gcc agc ctg gcg ctg acc ggg gct gtg gtg gct 462
cac gcc tac tac ctc aaa cac cag ttc tac ccc act gtg gtg tac ctg 510
acc aag tcc agc ccc agc atg gca gtc ctg tac atc cag gcc ttt gtc 558
ctt gtc ttc ctt ctg ggc aag gtg atg ggc aag gtg ttc ttt ggg caa 606
ctg agg gca gca gag atg gag cac ctt ctg gaa cgt tcc tgg tac gcc 654
gtc aca gag act tgt ctg gcc ttc acc gtt ttt cgg gat gac ttc agc 702
ccc cgc ttt gtt gca ctc ttc act ctt ctt ctc ttc ctc aaa tgt ttc 750
cac tgg ctg gct gag gac cgt gtg gac ttt atg gaa cgc agc ccc aac 798
atc tcc tgg ctc ttt cac tgc cgc att gtc tct ctt atg ttc ctc ctg 846
ggc atc ctg gac ttc ctc ttc gtc agc cac gcc tat cac agc atc ctg 894
acc cgt ggg gcc tct gtg cag ctg gtg ttt ggc ttt gag tat gcc atc 942
ctg atg acg atg gtg ctc acc atc ttc atc aag tat gtg ctg cac tcc 990
gtg gac ctc cag agt gag aac ccc tgg gac aac aag gct gtg tac atg 1038
ctc tac aca gag ctg ttt aca ggc ttc atc aag gtt ctg ctg tac atg 1086
gcc ttc atg acc atc atg atc aag gtg cac acc ttc cca ctc ttt gcc 1134
atc cgg ccc atg tac ctg gcc atg aga cag ttc aag aaa gct gtg aca 1182
gat gcc atc atg tct cgc cga gcc atc cgc aac atg aac acc ctg tat 1230
cca gat gcc acc cca gag gag ctc cag gca atg gac aat gtc tgc atc 1278
atc tgc cga gaa gag atg gtg act ggt gcc aag aga ctg ccc tgc aac 1326
cac att ttc cat acc agc tgc ctg cgc tcc tgg ttc cag cgg cag cag 1374
acc tgc ccc acc tgc cgt atg gat gtc ctt cgt gca tcg ctg cca gcg 1422
cag tca cca cca ccc ccg gag cct gcg gat cag ggg cca ccc cct gcc 1470
ccc cac ccc cca cca ctc ttg cct cag ccc ccc aac ttc ccc cag ggc 1518
ctc ctg cct cct ttt cct cca ggc atg ttc cca ctg tgg ccc ccc atg 1566
ggc ccc ttt cca cct gtc ccg cct ccc ccc agc tca gga gag gct gtg 1614
gct cct cca tcc acc agt gca gca gcc ctt tct cgg ccc agt gga gca 1662
gct aca acc aca gct gct ggc acc agt gct act gct gct tct gcc aca 1710
gca tct ggc cca ggc tct ggc tct gcc cca gag gct ggc cct gcc cct 1758
ggt ttc ccc ttc cct cct ccc tgg atg ggt atg ccc ctg cct cca ccc 1806
ttt gcc ttc ccc cca atg cct gtg ccc cct gcg ggc ttt gct ggg ctg 1854
acc cca gag gag cta cga gct ctg gag ggc cat gag cgg cag cac ctg 1902
gag gcc cgg ctg cag agc ctg cgt aac atc cac aca ctg ctg gac gcc 1950
gcc atg ctg cag atc aac cag tac ctc acc gtg ctg gcc tcc ttg ggg 1998
ccc ccc cgg cct gcc act tca gtc aac tcc act gag ggg act gcc act 2046
aca gtt gtt gct gct gcc tcc tcc acc agc atc cct agc tca gag gcc 2094
acg acc cca acc cca gga gcc tcc cca cca gcc cct gaa atg gaa agg 2142
cct cca gct cct gag tca gtg ggc aca gag gag atg cct gag gat gga 2190
gag ccc gat gca gca gag ctc cgc cgg cgc cgc ctg cag aag ctg gag 2238
tct cct gtt gcc cac tga cactgcccca gcccagcccc agcctctgct 2286
cttttgagca gccctcgctg gaacatgtcc tgccaccaag tgccagctcc ctctctgtct 2346
gcaccaggga gtagtacccc cagctctgag aaagaggcgg catcccctag gccaagtgga 2406
aagaggctgg ggttcccatt tgactccagt cccaggcagc catggggatc tcgggtcagt 2466
tccagccttc ctctccaact cttcagccct gtgttctgct ggggccatga aggcagaagg 2526
tttagcctct gagaagccct cttcttcccc cacccctttc caggagaagg ggctgcccct 2586
ccaagcccta cttgtatgtg cggagtcaca ctgcagtgcc gaacagtatt agctcccgtt 2646
cccaagtgtg gactccagag gggctggagg caagctatga acttgctcgc tggcccaccc 2706
ctaagactgg tacccatttc cttttcttac cctgatctcc ccagaagcct cttgtggtgg 2766
tggctgtgcc ccctatgccc tgtggcattt ctgcgtctta ctggcaacca cacaactcag 2826
ggaaaggaat gcctgggagt gggggtgcag gcgggcagca ctgagggacc ctgccccgcc 2886
cctcccccca ggcccctttc ccctgcagct tctcaagtga gactgacctg tctcacccag 2946
cagccactgc ccagccgcac tccaggcaag ggccagtgcg cctgctcctg accactgcaa 3006
tcccagcgcc caaggaaggc cacttctcaa ctggcagaac ttctgaagtt tagaattgga 3066
attacttcct tactagtgtc ttttggctta aattttgtct tttgaagttg aatgcttaat 3126
cccgggaaag aggaacagga gtgccagact cctggtcttt ccagtttaga aaaggctctg 3186
tgccaaggag ggaccacagg agctgggacc tgcctgcccc tgtcctttcc ccttggtttt 3246
gtgttacaag agttgttgga gacagtttca gatgattatt taatttgtaa atattgtaca 3306
aattttaata gcttaaattg tatatacagc caaataaaaa cttgcattaa caaaaaaaaa 3366
aaaaaaaa 3374

<210> 2

<211> 617

<212> PRT

<213> Homo sapiens

<400> 2
<210> 3

<211> 3028

<212> DNA

<213> Homo sapiens

<220>

<221> CDS

<222> (60)..(1910)

<400> 3

gcagtcgcgc ggattgagcg ggctcgcggc gctgggttcc tggtctccgg gccagggca 59

atg ttc cgc acg gca gtg atg atg gcg gcc agc ctg gcg ctg acc ggg 107

Met Phe Arg Thr Ala Val Met Met Ala Ala Ser Leu Ala Leu Thr Gly

1               5                   10                  15

gct gtg gtg gct cac gcc tac tac ctc aaa cac cag ttc tac ccc act 155

Ala Val Val Ala His Ala Tyr Tyr Leu Lys His Gln Phe Tyr Pro Thr

            20                  25                  30

gtg gtg tac ctg acc aag tcc agc ccc agc atg gca gtc ctg tac atc 203

Val Val Tyr Leu Thr Lys Ser Ser Pro Ser Met Ala Val Leu Tyr Ile

        35                  40                  45

cag gcc ttt gtc ctt gtc ttc ctt ctg ggc aag gtg atg ggc aag gtg 251

Gln Ala Phe Val Leu Val Phe Leu Leu Gly Lys Val Met Gly Lys Val

    50                  55                  60

ttc ttt ggg caa ctg agg gca gca gag atg gag cac ctt ctg gaa cgt 299
Phe Phe Gly Gln Leu Arg Ala Ala Glu Met Glu His Leu Leu Glu Arg
65                  70                  75                  80

tcc tgg tac gcc gtc aca gag act tgt ctg gcc ttc acc gtt ttt cgg 347
Ser Trp Tyr Ala Val Thr Glu Thr Cys Leu Ala Phe Thr Val Phe Arg
                85                  90                  95

gat gac ttc agc ccc cgc ttt gtt gca ctc ttc act ctt ctt ctc ttc 395
Asp Asp Phe Ser Pro Arg Phe Val Ala Leu Phe Thr Leu Leu Leu Phe
            100                 105                 110

ctc aaa tgt ttc cac tgg ctg gct gag gac cgt gtg gac ttt atg gaa 443
Leu Lys Cys Phe His Trp Leu Ala Glu Asp Arg Val Asp Phe Met Glu
        115                 120                 125

cgc agc ccc aac atc tcc tgg ctc ttt cac tgc cgc att gtc tct ctt 491
Arg Ser Pro Asn Ile Ser Trp Leu Phe His Cys Arg Ile Val Ser Leu
    130                 135                 140

atg ttc ctc ctg ggc atc ctg gac ttc ctc ttc gtc agc cac gcc tat 539
Met Phe Leu Leu Gly Ile Leu Asp Phe Leu Phe Val Ser His Ala Tyr
145                 150                 155                 160

cac agc atc ctg acc cgt ggg gcc tct gtg cag ctg gtg ttt ggc ttt 587

His Ser Ile Leu Thr Arg Gly Ala Ser Val Gln Leu Val Phe Gly Phe
                165                 170                 175

gag tat gcc atc ctg atg acg atg gtg ctc acc atc ttc atc aag tat 635
Glu Tyr Ala Ile Leu Met Thr Met Val Leu Thr Ile Phe Ile Lys Tyr
            180                 185                 190

gtg ctg cac tcc gtg gac ctc cag agt gag aac ccc tgg gac aac aag 683
Val Leu His Ser Val Asp Leu Gln Ser Glu Asn Pro Trp Asp Asn Lys
        195                 200                 205

gct gtg tac atg ctc tac aca gag ctg ttt aca ggc ttc atc aag gtt 731
Ala Val Tyr Met Leu Tyr Thr Glu Leu Phe Thr Gly Phe Ile Lys Val
    210                 215                 220

ctg ctg tac atg gcc ttc atg acc atc atg atc aag gtg cac acc ttc 779
Leu Leu Tyr Met Ala Phe Met Thr Ile Met Ile Lys Val His Thr Phe
225                 230                 235                 240

cca ctc ttt gcc atc cgg ccc atg tac ctg gcc atg aga cag ttc aag 827
Pro Leu Phe Ala Ile Arg Pro Met Tyr Leu Ala Met Arg Gln Phe Lys
                245                 250                 255

aaa gct gtg aca gat gcc atc atg tct cgc cga gcc atc cgc aac atg 875
Lys Ala Val Thr Asp Ala Ile Met Ser Arg Arg Ala Ile Arg Asn Met
            260                 265                 270

aac acc ctg tat cca gat gcc acc cca gag gag ctc cag gca atg gac 923
Asn Thr Leu Tyr Pro Asp Ala Thr Pro Glu Glu Leu Gln Ala Met Asp
        275                 280                 285

aat gtc tgc atc atc tgc cga gaa gag atg gtg act ggt gcc aag aga 971
Asn Val Cys Ile Ile Cys Arg Glu Glu Met Val Thr Gly Ala Lys Arg
    290                 295                 300

ctg ccc tgc aac cac att ttc cat acc agc tgc ctg cgc tcc tgg ttc 1019
Leu Pro Cys Asn His Ile Phe His Thr Ser Cys Leu Arg Ser Trp Phe
305                 310                 315                 320

cag cgg cag cag acc tgc ccc acc tgc cgt atg gat gtc ctt cgt gca 1067
Gln Arg Gln Gln Thr Cys Pro Thr Cys Arg Met Asp Val Leu Arg Ala
                325                 330                 335

tcg ctg cca gcg cag tca cca cca ccc ccg gag cct gcg gat cag ggg 1115
Ser Leu Pro Ala Gln Ser Pro Pro Pro Pro Glu Pro Ala Asp Gln Gly
            340                 345                 350

cca ccc cct gcc ccc cac ccc cca cca ctc ttg cct cag ccc ccc aac 1163
Pro Pro Pro Ala Pro His Pro Pro Pro Leu Leu Pro Gln Pro Pro Asn
        355                 360                 365

ttc ccc cag ggc ctc ctg cct cct ttt cct cca ggc atg ttc cca ctg 1211

Phe Pro Gln Gly Leu Leu Pro Pro Phe Pro Pro Gly Met Phe Pro Leu
    370                 375                 380

tgg ccc ccc atg ggc ccc ttt cca cct gtc ccg cct ccc ccc agc tca 1259
Trp Pro Pro Met Gly Pro Phe Pro Pro Val Pro Pro Pro Pro Ser Ser
385                 390                 395                 400

gga gag gct gtg gct cct cca tcc acc agt gca gcc ctt tct cgg ccc 1307
Gly Glu Ala Val Ala Pro Pro Ser Thr Ser Ala Ala Leu Ser Arg Pro
                405                 410                 415

agt gga gca gct aca acc aca gct gct ggc acc agt gct act gct gct 1355
Ser Gly Ala Ala Thr Thr Thr Ala Ala Gly Thr Ser Ala Thr Ala Ala
            420                 425                 430

tct gcc aca gca tct ggc cca ggc tct ggc tct gcc cca gag gct ggc 1403
Ser Ala Thr Ala Ser Gly Pro Gly Ser Gly Ser Ala Pro Glu Ala Gly
        435                 440                 445

cct gcc cct ggt ttc ccc ttc cct cct ccc tgg atg ggt atg ccc ctg 1451
Pro Ala Pro Gly Phe Pro Phe Pro Pro Pro Trp Met Gly Met Pro Leu
    450                 455                 460

cct cca ccc ttt gcc ttc ccc cca atg cct gtg ccc cct gcg ggc ttt 1499
Pro Pro Pro Phe Ala Phe Pro Pro Met Pro Val Pro Pro Ala Gly Phe
465                 470                 475                 480

gct ggg ctg acc cca gag gag cta cga gct ctg gag ggc cat gag cgg 1547
Ala Gly Leu Thr Pro Glu Glu Leu Arg Ala Leu Glu Gly His Glu Arg
                485                 490                 495

cag cac ctg gag gcc cgg ctg cag agc ctg cgt aac atc cac aca ctg 1595
Gln His Leu Glu Ala Arg Leu Gln Ser Leu Arg Asn Ile His Thr Leu
            500                 505                 510

ctg gac gcc gcc atg ctg cag atc aac cag tac ctc acc gtg ctg gcc 1643
Leu Asp Ala Ala Met Leu Gln Ile Asn Gln Tyr Leu Thr Val Leu Ala
        515                 520                 525

tcc ttg ggg ccc ccc cgg cct gcc act tca gtc aac tcc act gag ggg 1691
Ser Leu Gly Pro Pro Arg Pro Ala Thr Ser Val Asn Ser Thr Glu Gly
    530                 535                 540

act gcc act aca gtt gtt gct gct gcc tcc tcc acc agc atc cct agc 1739
Thr Ala Thr Thr Val Val Ala Ala Ala Ser Ser Thr Ser Ile Pro Ser
545                 550                 555                 560

tca gag gcc acg acc cca acc cca gga gcc tcc cca cca gcc cct gaa 1787
Ser Glu Ala Thr Thr Pro Thr Pro Gly Ala Ser Pro Pro Ala Pro Glu
                565                 570                 575

atg gaa agg cct cca gct cct gag tca gtg ggc aca gag gag atg cct 1835

Met Glu Arg Pro Pro Ala Pro Glu Ser Val Gly Thr Glu Glu Met Pro
            580                 585                 590

gag gat gga gag ccc gat gca gca gag ctc cgc cgg cgc cgc ctg cag 1883
Glu Asp Gly Glu Pro Asp Ala Ala Glu Leu Arg Arg Arg Arg Leu Gln
        595                 600                 605

aag ctg gag tct cct gtt gcc cac tga cactgcccca gcccagcccc 1930
Lys Leu Glu Ser Pro Val Ala His
    610                 615

agcctctgct cttttgagca gccctcgctg gaacatgtcc tgccaccaag tgccagctcc 1990

ctctctgtct gcaccaggga gtagtacccc cagctctgag aaagaggcgg catcccctag 2050

gccaagtgga aagaggctgg ggttcccatt tgactccagt cccaggcagc catggggatc 2110

tcgggtcagt tccagccttc ctctccaact cttcagccct gtgttctgct ggggccatga 2170

aggcagaagg tttagcctct gagaagccct cttcttcccc cacccctttc caggagaagg 2230

ggctgcccct ccaagcccta cttgtatgtg cggagtcaca ctgcagtgcc gaacagtatt 2290

agctcccgtt cccaagtgtg gactccagag gggctggagg caagctatga acttgctcgc 2350

tggcccaccc ctaagactgg tacccatttc cttttcttac cctgatctcc ccagaagcct 2410

cttgtggtgg tggctgtgcc ccctatgccc tgtggcattt ctgcgtctta ctggcaacca 2470

cacaactcag ggaaaggaat gcctgggagt gggggtgcag gcgggcagca ctgagggacc 2530

ctgccccgcc cctcccccca ggcccctttc ccctgcagct tctcaagtga gactgacctg 2590

tctcacccag cagccactgc ccagccgcac tccaggcaag ggccagtgcg cctgctcctg 2650

accactgcaa tcccagcgcc caaggaaggc cacttctcaa ctggcagaac ttctgaagtt 2710

tagaattgga attacttcct tactagtgtc ttttggctta aattttgtct tttgaagttg 2770

aatgcttaat cccgggaaag aggaacagga gtgccagact cctggtcttt ccagtttaga 2830

aaaggctctg tgccaaggag ggaccacagg agctgggacc tgcctgcccc tgtcctttcc 2890

ccttggtttt gtgttacaag agttgttgga gacagtttca gatgattatt taatttgtaa 2950

atattgtaca aattttaata gcttaaattg tatatacagc caaataaaaa cttgcattaa 3010

caaaaaaaaa aaaaaaaa 3028

<210> 4

<211> 616

<212> PRT

<213> Homo sapiens

<400> 4

Met Phe Arg Thr Ala Val Met Met Ala Ala Ser Leu Ala Leu Thr Gly
1               5                   10                  15

Ala Val Val Ala His Ala Tyr Tyr Leu Lys His Gln Phe Tyr Pro Thr
            20                  25                  30

Val Val Tyr Leu Thr Lys Ser Ser Pro Ser Met Ala Val Leu Tyr Ile
        35                  40                  45

Gln Ala Phe Val Leu Val Phe Leu Leu Gly Lys Val Met Gly Lys Val
    50                  55                  60

Phe Phe Gly Gln Leu Arg Ala Ala Glu Met Glu His Leu Leu Glu Arg
65                  70                  75                  80

Ser Trp Tyr Ala Val Thr Glu Thr Cys Leu Ala Phe Thr Val Phe Arg
                85                  90                  95

Asp Asp Phe Ser Pro Arg Phe Val Ala Leu Phe Thr Leu Leu Leu Phe
            100                 105                 110

Leu Lys Cys Phe His Trp Leu Ala Glu Asp Arg Val Asp Phe Met Glu

        115                 120                 125

Arg Ser Pro Asn Ile Ser Trp Leu Phe His Cys Arg Ile Val Ser Leu
    130                 135                 140

Met Phe Leu Leu Gly Ile Leu Asp Phe Leu Phe Val Ser His Ala Tyr
145                 150                 155                 160

His Ser Ile Leu Thr Arg Gly Ala Ser Val Gln Leu Val Phe Gly Phe
                165                 170                 175

Glu Tyr Ala Ile Leu Met Thr Met Val Leu Thr Ile Phe Ile Lys Tyr
            180                 185                 190

Val Leu His Ser Val Asp Leu Gln Ser Glu Asn Pro Trp Asp Asn Lys
        195                 200                 205

Ala Val Tyr Met Leu Tyr Thr Glu Leu Phe Thr Gly Phe Ile Lys Val
    210                 215                 220

Leu Leu Tyr Met Ala Phe Met Thr Ile Met Ile Lys Val His Thr Phe
225                 230                 235                 240

Pro Leu Phe Ala Ile Arg Pro Met Tyr Leu Ala Met Arg Gln Phe Lys
                245                 250                 255

Lys Ala Val Thr Asp Ala Ile Met Ser Arg Arg Ala Ile Arg Asn Met
            260                 265                 270
Asn Thr Leu Tyr Pro Asp Ala Thr Pro Glu Glu Leu Gln Ala Met Asp
        275                 280                 285
Asn Val Cys Ile Ile Cys Arg Glu Glu Met Val Thr Gly Ala Lys Arg
    290                 295                 300
Leu Pro Cys Asn His Ile Phe His Thr Ser Cys Leu Arg Ser Trp Phe
305                 310                 315                 320
Gln Arg Gln Gln Thr Cys Pro Thr Cys Arg Met Asp Val Leu Arg Ala
                325                 330                 335
Ser Leu Pro Ala Gln Ser Pro Pro Pro Pro Glu Pro Ala Asp Gln Gly
            340                 345                 350
Pro Pro Pro Ala Pro His Pro Pro Pro Leu Leu Pro Gln Pro Pro Asn
        355                 360                 365
Phe Pro Gln Gly Leu Leu Pro Pro Phe Pro Pro Gly Met Phe Pro Leu
    370                 375                 380
Trp Pro Pro Met Gly Pro Phe Pro Pro Val Pro Pro Pro Pro Ser Ser
385                 390                 395                 400

Gly Glu Ala Val Ala Pro Pro Ser Thr Ser Ala Ala Leu Ser Arg Pro
                405                 410                 415
Ser Gly Ala Ala Thr Thr Thr Ala Ala Gly Thr Ser Ala Thr Ala Ala
            420                 425                 430
Ser Ala Thr Ala Ser Gly Pro Gly Ser Gly Ser Ala Pro Glu Ala Gly
        435                 440                 445
Pro Ala Pro Gly Phe Pro Phe Pro Pro Pro Trp Met Gly Met Pro Leu
    450                 455                 460
Pro Pro Pro Phe Ala Phe Pro Pro Met Pro Val Pro Pro Ala Gly Phe
465                 470                 475                 480
Ala Gly Leu Thr Pro Glu Glu Leu Arg Ala Leu Glu Gly His Glu Arg
                485                 490                 495
Gln His Leu Glu Ala Arg Leu Gln Ser Leu Arg Asn Ile His Thr Leu
            500                 505                 510
Leu Asp Ala Ala Met Leu Gln Ile Asn Gln Tyr Leu Thr Val Leu Ala
        515                 520                 525
Ser Leu Gly Pro Pro Arg Pro Ala Thr Ser Val Asn Ser Thr Glu Gly

    530                 535                 540

Thr Ala Thr Thr Val Val Ala Ala Ala Ser Ser Thr Ser Ile Pro Ser
545                 550                 555                 560

Ser Glu Ala Thr Thr Pro Thr Pro Gly Ala Ser Pro Pro Ala Pro Glu
                565                 570                 575

Met Glu Arg Pro Pro Ala Pro Glu Ser Val Gly Thr Glu Glu Met Pro
            580                 585                 590

Glu Asp Gly Glu Pro Asp Ala Ala Glu Leu Arg Arg Arg Arg Leu Gln
        595                 600                 605

Lys Leu Glu Ser Pro Val Ala His
    610                 615

## Description

### Technical Field

The present invention relates to the utilization of synoviolin, which is isolated as a synovial cell protein, and its gene. Specifically, the present invention relates to their utilization in therapeutic formulations and therapeutic methods for hematopoietic diseases.

### Background Art

Quality control of proteins in endoplasmic reticulum (ER) and transcriptional control of the amount of proteins in the nucleus are important processes that maintain cellular homeostasis (Hampton, R.Y., 2002, "ER-associated degradation in protein quality control and cellular regulation." Curr. Opin. Cell Biol. 14: 476-482). In eukaryotic cells, newly synthesized proteins are transported into the ER where they are correctly folded. However, various environmental conditions, such as large amounts of protein influx into the ER, could trigger a cellular response called unfolded protein response (URP) to overcome this problem (Welihinda, A.A., Tirasophon, W., and Kaufman, R.J., 1999, "The cellular response to protein misfolding in the endoplasmic reticulum." Gene Expr. 7: 293-300). During the URP response, synthesis of new proteins is globally inhibited by inactivation of eukaryotic initiation factor (eIF) 2α to reduce additional accumulation of misfolded proteins in ER, and genes encoding the ER chaperone proteins including Bip/Grp78 and Grp94, are also upregulated to re-fold the misfolded proteins correctly (Ron, D., 2002, "Translational control in the endoplasmic reticulum stress response." J. Clin. Invest. 110: 1383-1388). When the amount of misfolded proteins exceeds the protein folding capacity, in spite of URP, misfolded proteins are eliminated by ubiquitin- and proteasome-dependent degradation processes, known as ER-associated degradation (ERAD) (Hampton, 2002, supra). Misfolded proteins in the ER are translocated into the cytosol, where they become targets of 26S proteasome by ubiquitin ligase enzymes. Various ubiquitin ligases are reported in the ERAD system in mammalian cells, including CHIP (C-terminus of Hsc70-interacting protein) (Ballinger, C.A., Connell, P., Wu, Y., Hu, Z., Thompson, L.J., Yin, L.Y., and Patterson, C., 1999, "Identification of CHIP, a novel tetratricopeptide repeat-containing protein that interacts with heat shock proteins and negatively regulates chaperone functions." Mol. Cell Biol. 19: 4535-4545; Meacham, G.C., Patterson, C., Zhang, W., Younger, J.M., and Cyr, D.M., 2001, "The Hsc70 co-chaperone CHIP targets immature CFTR for proteasomal degradation." Nat. Cell Biol. 3: 100-105; Imai, Y, Soda, M., Inoue, H., Hattori, N., Mizuno, Y, and Takahashi, R., 2001, "An Unfolded Putative Transmembrane Polypeptide, which Can Lead Endoplasmic Reticulum Stress, Is a Substrate of Parkin." Cell 105: 891-902), parkin (Imai, Y, Soda, M., Hatakeyama, S., Akagi, T., Hashikawa, T., Nakayama, K.I., and Takahashi, R., 2002, "CHIP is associated with Parkin, a gene responsible for familial Parkinson's disease, and enhances its ubiquitin ligase activity." Mol. Cell 10: 55-67), gp78/AMFR (Shimizu, K., Tani, M., Watanabe, H., Nagamachi, Y, Niinaka, Y., Shiroishi, T., Ohwada, S., Raz, A., and Yokota, J., 1999, "The autocrine motility factor receptor gene encodes a novel type of seven transmembrane protein." FEBS Lett. 456: 295-300; Fang, S., Ferrone, M., Yang, C., Jensen, J.P., Tiwari, S., and Weissman, A.M., 2001, "The tumor autocrine motility factor receptor, gp78, is a ubiquitin protein ligase implicated in degradation from the endoplasmic reticulum." Proc. Natl. Acad. Sci. U.S.A. 98: 14422-14427) and Fbx2/FBG1/NFB42 (Yoshida, Y, Chiba, T., Tokunaga, F., Kawasaki, H., Iwai, K., Suzuki, T., Ito, Y, Matsuoka, K., Yoshida, M., Tanaka, K., and Tai, T., 2002, "E3 ubiquitin ligase that recognizes sugar chains." Nature 418: 438-442). Intensive research is currently being conducted to determine the precise mechanisms that regulate the ERAD system in the ER.

It is reported that the ERAD system constitutively functions to eliminate the amount of misfolded proteins produced during cell growth (Travers, K.J., Patil, C.K., Wodicka, L., Lockhart, D.J., Weissman, J.S., and Walter, P., 2000, "Functional and genomic analyses reveal an essential coordination between the unfolded protein response and ER-associated degradation." Cell 101: 249-258). Recent studies have shown that functional disruption of the UPR and/or the ERAD system can augment caspase-dependent apoptosis of cells treated with some ER stress inducible chemical agents (Nakagawa, T., Zhu, H., Morishima, N., Li, E., Xu, J., Yankner, B.A., and Yuan, J., 2000, "Caspase-12 mediates endoplasmic-reticulum-specific apoptosis and cytotoxicity by amyloid-beta." Nature 403: 98-103), which are known to disturb proper protein folding in ER (Lee, A.S., 2001, "The glucose-regulated proteins: stress induction and clinical applications." Trends Biochem. Sci. 26: 504-510). These results can explain the molecular pathogenesis of certain human diseases that arise from ERAD system disruption. For instance, production of expanded polyglutamine causes certain inherited neurodegenerative disorders (Jana, N.R., Zemskov, E.A., Wang, Gh., and Nukina, N., 2001, "Altered proteasomal function due to the expression of polyglutamine-expanded truncated N-terminal huntingtin induces apoptosis by caspase activation through mitochondrial cytochrome c release." Hum. Mol. Genet. 10: 1049-1059; Bence, N.F., Sampat, R.M., and Kopito, R.R., 2001, "Impairment of the ubiquitin-proteasome system by protein aggregation." Science 292: 1552-1555; Hirabayashi, M., Inoue, K., Tanaka, K., Nakadate, K., Ohsawa, Y., Kamei, Y., Popiel, A.H., Sinohara, A., Iwamatsu, A., Kimura, Y., Uchiyama, Y., Hori, S., and Kakizuka, A., 2001, "VCP/p97 in abnormal protein aggregates, cytoplasmic vacuoles, and cell death, phenotypes relevant to neurodegeneration." Cell Death. Differ. 8: 977-984). Alternatively, mutations in the parkin gene, a famous ubiquitin ligase protein in ERAD system, are thought to result in neuronal cell death of the substantia nigra in patients with autosomal recessive juvenile parkinsonism (AR-JP) (Imai, Y, Soda, M., and Takahashi, R., 2000, "Parkin suppresses unfolded protein stress-induced cell death through its E3 ubiquitin-protein ligase activity." J. Biol. Chem. 275: 35661-35664). These findings emphasize the importance of the ERAD system in cell survival under both physiological and pathological conditions.

Recently, by immunoscreening using anti-synovial cell antibodies, the present inventors cloned synoviolin/HRD 1 (herein after abbreviated as "synoviolin"), a human homologue of yeast ubiquitin ligase (E3) Hrdlp/Del3 (Bays, N.W., Gardner, R.G., Seelig, L.P., Joazeiro, C.A., and Hampton, R.Y., 2001, "Hrd1p/Der3p is a membrane-anchored ubiquitin ligase required for ER-associated degradation." Nat. Cell Biol. 3: 24-29), which is an ER resident membrane protein that comprises a RING-H2 motif.

Synoviolin was obtained from immunoscreening using anti-synovial cell antibodies as described above. Synoviolin is suggested to be heavily involved in the onset of rheumatoid arthritis (hereinafter abbreviated as "RA"). In spite that synoviolin is, in fact, hardly expressed in the synovial tissues of osteoarthritis patients, strong expression is apparent in the synovial tissues of RA patients. There are reports that mice overexpressing the synoviolin gene develop synovial hyperplasia and rheumatism-like symptoms accompanied by cartilage and bone destruction, and this supports the presumption that synoviolin is involved in RA.

On the other hand, Hrd1p, the yeast homologue of synoviolin, is known to be one of the molecules that remove structurally abnormal proteins produced and accumulated in the endoplasmic reticulum due to stress, such as ischemia, hypoxia, heat shock, amino acid starvation, viral infection, and decrease of endoplasmic reticulum lumenal calcium concentration, and prevent rupture of the endoplasmic reticulum. The endoplasmic reticulum stress response mechanisms are consisted of three mechanisms: (1) transcriptional induction of folding enzymes and chaperone molecules in the endoplasmic reticulum, (2) suppression of protein translation, and (3) active degradation of abnormal proteins by the ubiquitin-proteasome system. Generally, (1) and (2) are referred to as the unfolded protein response (UPR), and (3) is referred to as the endoplasmic reticulum-associated degradation (ERAD), where yeast Hrd1p is involved. When cells receive excessive stress which is beyond the capacity of crisis management mechanisms - UPR and ERAD, the cells themselves choose to undergo apoptosis. Similarly, the collapse of ERAD leads to apoptosis.

Since synoviolin also has E3 activity and is expressed at the ER in cells, like yeast Hrd1p, it is involved in ERAD and takes part in the endoplasmic reticulum stress response mechanism.

### Disclosure of the Invention

The present inventors generated synoviolin knockout mice lacking synoviolin, which is a protein considered to function as a quality control ligase and participate in ERAD - one of the endoplasmic reticulum stress response mechanisms, and attempted to elucidate the *in vivo* mechanism of the protein.

Analyses showed that the synoviolin knockout mice died as fetuses at E12.5-13.5. Detailed investigation of the fetal tissues for understanding the cause of death revealed increased apoptosis in the liver and a resultant decrease in cell density. To one's surprise, deleting the function of synoviolin, which is one of many existing ubiquitin ligases, led to lethality in mice at the developmental stage, thus making this an absolutely novel and totally unexpected finding. Further examination of the synoviolin knockout mice showed that these mice have abnormalities in their hematopoietic system, and hematophagocytosis is increased due to activation of macrophages. More specifically, the examination proved that one of the consequences of augmented apoptosis in the synoviolin knockout mice is abnormal erythroblast differentiation, which activates macrophages to remove the resultantly increased abnormal erythroblasts. This causes anemia as a result of the decreased number of blood cells, and the mice die at this stage. Examinations using mouse embryonic fibroblasts (hereinafter abbreviated as "MEFs") revealed that this augmentation of apoptosis was induced by ER stress, as expected. In other words, this result suggests that the ER stress strongly affects secondary hematopoiesis during mouse development, and provides a completely novel finding that failure of ERAD causes lethality in mice. As mentioned earlier, the present study demonstrated for the first time that a factor called synoviolin, among ubiquitin ligases, plays a particularly important role in ERAD.

The present inventors have previously reported the involvement of synoviolin in the onset of RA; therefore, targeting the function of synoviolin which plays an important role in ERAD is considered to provide treatment for this disease. Furthermore, therapeutic effects and such are predicted for not only hematopoietic disorders and RA, but also diseases related to ER stress-induced apoptosis. Thus, the present study is expected to lead to the development of new pharmaceuticals.

Based on the above-mentioned findings, the present invention provides the following therapeutic pharmaceutical agents and therapeutic methods for hematopoietic diseases, and methods of screening for pharmaceutical agents for treatment of hematopoietic diseases.
[1] A method for treating hematopoietic diseases, wherein the method comprises administering any one of the proteins selected from the group consisting of (a) to (d) shown below, or a polynucleotide encoding the protein:
   (a) a protein comprising the amino acid sequence of SEQ ID NO: 2;
   (b) a protein comprising an amino acid sequence with one or more amino acid substitutions, deletions, insertions, and/or additions in the amino acid sequence of SEQ ID NO: 2, wherein the protein is functionally equivalent to a protein comprising the amino acid sequence of SEQ ID NO: 2;
   (c) a protein encoded by a polynucleotide that hybridizes under stringent conditions with a polynucleotide comprising the nucleotide sequence of SEQ ID NO: 1, wherein the protein is functionally equivalent to a protein comprising the amino acid sequence of SEQ ID NO: 2; and
   (d) a protein encoded by a polynucleotide comprising a nucleotide sequence with at least 70% or higher homology to the nucleotide sequence of SEQ ID NO: 1, wherein the protein is functionally equivalent to a protein comprising the amino acid sequence of SEQ ID NO: 2.
[2] The method of [1], wherein the hematopoietic diseases are diseases caused by abnormal erythroblast differentiation.
[3] The method of [1], which comprises introducing into hematopoietic stem cells a vector harboring the polynucleotide in an expressible state.
[4] A method for inducing erythroblast differentiation, wherein the method comprises expressing in hematopoietic stem cells any one of the proteins selected from the group consisting of (a) to (d) shown below:
   (a) a protein comprising the amino acid sequence of SEQ ID NO: 2;
   (b) a protein comprising an amino acid sequence with one or more amino acid substitutions, deletions, insertions, and/or additions in the amino acid sequence of SEQ ID NO: 2, wherein the protein is functionally equivalent to a protein comprising the amino acid sequence of SEQ ID NO: 2;
   (c) a protein encoded by a polynucleotide that hybridizes under stringent conditions with a polynucleotide comprising the nucleotide sequence of SEQ ID NO: 1, wherein the protein is functionally equivalent to a protein comprising the amino acid sequence of SEQ ID NO: 2; and
   (d) a protein encoded by a polynucleotide comprising a nucleotide sequence with at least 70% or higher homology to the nucleotide sequence of SEQ ID NO: 1, wherein the protein is functionally equivalent to a protein comprising the amino acid sequence of SEQ ID NO: 2.
[5] A pharmaceutical formulation for treating hematopoietic diseases, wherein the formulation comprises as an effective ingredient any one of the proteins selected from the group consisting of (a) to (d) shown below, or a polynucleotide encoding the protein:
   (a) a protein comprising the amino acid sequence of SEQ ID NO: 2;
   (b) a protein comprising an amino acid sequence with one or more amino acid substitutions, deletions, insertions, and/or additions in the amino acid sequence of SEQ ID NO: 2, wherein the protein is functionally equivalent to a protein comprising the amino acid sequence of SEQ ID NO: 2;
   (c) a protein encoded by a polynucleotide that hybridizes under stringent conditions with a polynucleotide comprising the nucleotide sequence of SEQ ID NO: 1, wherein the protein is functionally equivalent to a protein comprising the amino acid sequence of SEQ ID NO: 2; and
   (d) a protein encoded by a polynucleotide comprising a nucleotide sequence with at least 70% or higher homology to the nucleotide sequence of SEQ ID NO: 1, wherein the protein is functionally equivalent to a protein comprising the amino acid sequence of SEQ ID NO: 2.
[6] The pharmaceutical formulation of [5], which is a hematopoietic stem cell harboring the polynucleotide in an expressible state.
[7] A method for treating hematopoietic diseases, wherein the method comprises administering an agent that enhances the activity of a protein comprising the amino acid sequence of SEQ ID NO: 2.
[8] A therapeutic agent for hematopoietic diseases, which comprises an agent that enhances the activity of a protein comprising the amino acid sequence of SEQ ID NO: 2 as an effective ingredient.
[9] A non-human hematopoietic disease model animal embryo whose synoviolin gene function is defective.
[10] The non-human hematopoietic disease model animal embryo of [9], wherein the non-human animal is a rodent.
[11] The non-human hematopoietic disease model animal embryo of [10], wherein the rodent is a mouse.
[12] The non-human hematopoietic disease model animal embryo of [11], which is an embryo of E 13.5 or younger.
[13] The non-human hematopoietic disease model animal embryo of [9], wherein the hematopoietic disease is a disease caused by abnormal erythroblast differentiation.
[14] A hematopoietic disease model cell derived from the non-human hematopoietic disease model animal embryo of any one of [9] to [13].
[15] The hematopoietic disease model cell of [14], which is a fibroblast.
[16] A method of screening for therapeutic agents for hematopoietic diseases, wherein the method comprises administering a test substance to a non-human hematopoietic disease model animal embryo whose synoviolin gene function is defective, and evaluating the condition of erythroblasts in the non-human animal embryo.
[17] A method of screening for therapeutic agents for hematopoietic diseases, wherein the method comprises the steps of:
   (a) administering or contacting an ER stress-inducing agent with a non-human hematopoietic disease model animal embryos whose synoviolin gene function is defective, or a cell derived from the embryos;
   (b) administering or contacting a test substance to the hematopoietic disease model non-human animal embryos whose synoviolin gene function is defective, or cells derived from the embryos, before, after, or simultaneously with step (a); and
   (c) determining cells in which apoptosis was induced from among the non-human animal embryos or the cells derived from the embryos.
[18] A method of screening for ER stress-removing agents, wherein the method includes the steps of:
   (a) acting an ER stress-inducing agent on cells whose endogenous synoviolin gene function is defective;
   (b) contacting a test substance to the cells before, after, or simultaneously with step (a); and
   (c) determining cells in which apoptosis was induced from among the cells.

First of all, the present invention provides methods for treating hematopoietic diseases, which utilize the synoviolin protein or a polynucleotide encoding it. Studies using synoviolin knockout mice have demonstrated that synoviolin is involved in hematopoiesis, particularly in the process of erythroblast differentiation. Therefore, the methods of the present invention may become effective therapeutic methods for hematopoietic diseases, in particular, diseases caused by abnormalities in the erythroblast differentiation process, such as anemia.

Examples of a synoviolin protein that can be used in the methods of the present invention include a protein comprising the amino acid sequence of SEQ ID NO: 2. This protein is encoded by the protein coding region of the nucleotide sequence shown in SEQ ID NO: 1. A synoviolin-encoding polynucleotide can be cloned by known methods (Nucleic Acid Res. 16:7583-7600, 1988). The present inventors discovered that synoviolin is strongly expressed in the synovial cells of RA patients; therefore, synoviolin gene can be isolated from a cDNA library obtained based on mRNAs extracted from synovial cells derived from tissues that have developed arthritis in RA patients (Nucleic Acid Research, 16, 7583, 1988), by using probes designed based on the nucleotide sequence of SEQ ID NO: 1 to screen for hybridizing clones. The synoviolin protein may be isolated and purified from the synovial cells of RA patients using substances having affinity to synoviolin such as synoviolin antibodies and synoviolin ligands (SL(S1-5), and such) (Lecka-Czernik, B. *et al.,* Mol. Cell. Biol. 15, 120-128, 1995; Heon, E. *et al.*, Arch. Ophthalmol. 114, 193-198, 1996; Ikegawa, S. *et al.,* Genomics 3 5, 590-592, 1996; Katsanis, N*. et al.,* Hum. Genet. 106, 66-72, 2000; Giltay, R*. et al.,* Matrix Biol. 18, 469-480, 1999; Stone, E. M. *et al.,* Nat. Genet. 22, 199-202, 1999). Alternatively, the synoviolin protein may be synthesized by joining the nucleotide sequence of SEQ ID NO: 1 to an expression vector and expressing the protein in suitable host cells.

Synoviolin protein in the present invention is not limited to the synoviolin protein comprising the sequence of SEQ ID NO: 2 described above, and also includes proteins that are functionally equivalent to this protein. In the present invention, for the synoviolin function, the proteins need to have a function of preventing hematopoietic cell apoptosis due to ER stress.

Examples of proteins that are functionally equivalent to synoviolin include (1) a protein comprising an amino acid sequence with one or more amino acid substitutions, deletions, insertions, and/or additions in the amino acid sequence of SEQ ID NO: 2; (2) a protein encoded by a polynucleotide that hybridizes under stringent conditions with a polynucleotide comprising the nucleotide sequence of SEQ ID NO: 1; and (3) a protein encoded by a polynucleotide comprising a nucleotide sequence with at least 70% or higher homology to the nucleotide sequence of SEQ ID NO: 1.

The number of non-homologous amino acids in these synoviolin-functionally equivalent proteins when compared to the synoviolin protein of SEQ ID NO: 2 is generally 50 amino acids or less, preferably 30 amino acids or less, and more preferably 5 amino acids or less (for example, 1 amino acid).

When obtaining synoviolin-functionally equivalent proteins by artificial amino acid substitution, the activity of the original protein may be easily maintained if the amino acids are substituted with amino acids of similar property. Conservative substitution may be important when substituting amino acids in domains that are important for protein activity. Conservative substitution of such amino acids is well known to those skilled in the art.

Groups of corresponding amino acids for conservative substitution include basic amino acids (for example, lysine, arginine, and histidine), acidic amino acids (for example, aspartic acid, and glutamic acid), uncharged polar amino acids (for example, glycine, asparagine, glutamine, serine, threonine, tyrosine, and cysteine), non-polar amino acids (for example, alanine, valine, leucine, isoleucine, proline, phenylalanine, methionine, and tryptophan), β-branched amino acids (for example, threonine, valine, and isoleucine), and aromatic amino acids (for example, tyrosine, phenylalanine, tryptophan, and histidine).

Activity of the protein may be increased (including for example, constitutively activated protein) or decreased by non-conservative substitution. As long as the protein has activity to rescue hematopoietic cells from apoptosis, even if its activity is decreased, the protein is included in the functionally equivalent proteins.

Examples of a method for obtaining synoviolin-functionally equivalent proteins include methods that utilize hybridization. More specifically, polynucleotides encoding the synoviolin of the present invention indicated by SEQ ID NO: 1, or fragments thereof, are used as probes, and polynucleotides that hybridize to them are isolated. By performing the hybridization under stringent conditions, polynucleotides having highly homologous nucleotide sequences will be selected, and as a result, the probability of synoviolin-functionally equivalent proteins be included in the isolated proteins is increased. Highly homologous nucleotide sequences refer to, for example, sequences with 70% or greater sequence identity, and desirably 90% or greater sequence identity.

The stringent conditions specifically refer to conditions such as 6x SSC, 40% formamide, hybridization at 25°C, and washing with 1x SSC at 55°C. Stringency depends on parameters such as salt concentration, formamide concentration, and temperature, and those skilled in the art can readily set these parameters so that the required stringency is achieved.

For example, a polynucleotide encoding a non-human synoviolin homologue can be isolated by utilizing hybridization. Homologues of synoviolin encoded by polynucleotides obtainable from a non-human animal species, i.e., mouse, rat, rabbit, pig, goat, or such, constitute functionally equivalent proteins of the present invention.

The present inventors cloned the synoviolin genes of the present invention from a number of individuals, and determined their nucleotide sequences to obtain a clone in which a single amino acid has been deleted. Proteins comprising mutations in their amino acid sequences and polynucleotides comprising a nucleotide sequence encoding such proteins are included in the synoviolin-functionally equivalent proteins and polynucleotides encoding such proteins of the present invention. The nucleotide sequence of the single amino acid-deficient clone confirmed by the present inventors is shown in SEQ ID NO: 3, and the amino acid sequence encoded by this nucleotide sequence is shown in SEQ ID NO: 4. The nucleotide sequence of SEQ ID NO: 3 lacks gca, which corresponds to positions 1293-1295 in SEQ ID NO: 1. As a result, the amino acid sequence shown in SEQ ID NO: 4 lacks Ala at position 412 of SEQ ID NO: 2.

Proteins obtained by introducing mutations into human synoviolin (SEQ ID NO: 2) and proteins encoded by polynucleotides isolated by the above hybridization techniques generally have a high amino acid sequence homology with human synoviolin (SEQ ID NO: 2). The "high homology" refers to a sequence identity of 30% or more, preferably 50% or more, and more preferably 80% or more (for example, 95% or more). The nucleotide sequence or amino acid sequence identity can be determined by using an internet homology search site [for example, homology searches such as FASTA, BLAST, PSI-BLAST, and SSEARCH in DNA Data Bank of Japan (DDBJ) can be used (for example, a homology search (Search and Analysis) page on the website of DNA Data Bank of Japan (DDBJ);
http://www.ddbj.nig.ac.jp/E-mail/homology-j.html). In addition, a search using BLAST can be carried out at the National Center for Biotechnology Information (NCBI) (for example, a BLAST page on the website of NCBI; http://www.ncbi.nlm.nih.gov/BLAST/; Altschul, S.F. *et al.,* J. Mol. Biol., 1990, 215(3):403-10; Altschul, S.F. & Gish, W., Meth. Enzymol., 1996, 266:460-480; Altschul, S.F. *et al.,* Nucleic Acids Res., 1997, 25:3389-3402)].

For example, the amino acid sequence identity can be calculated in Advanced BLAST 2.1 by using blastp as the program, setting the Expect value to 10, setting all Filters to OFF, using BLOSUM62 as the Matrix, setting the Gap existence cost, Per residue gap cost and Lambda ratio to 11, 1 and 0.85 (default values), respectively, and performing a search. The identity value (%) can then be obtained (Karlin, S. and S. F. Altschul (1990) Proc. Natl. Acad. Sci. USA 87:2264-2268; Karlin, S. and S. F. Altschul (1993) Proc. Natl. Acad. Sci. USA 90:5873-5877).

Examples of a method for confirming whether the synoviolin-functionally equivalent protein actually has functions equivalent to synoviolin include the complementarity experiments described in the Examples of the present application. More specifically, functional equivalence is confirmed by treating MEFs derived from homologous knockout embryos of synoviolin with ER stress agents, administering a DNA encoding the functionally equivalent protein described above, and then observing the inhibition of ER stress agent-induced apoptosis as an indicator.

In the methods of the present invention for treating hematopoietic diseases, polynucleotides encoding synoviolin or proteins functionally equivalent to synoviolin may be used. The polynucleotides may be of any origin. That is, they can be obtained from cDNA, or genomic DNA, or by synthesis. When using the polynucleotides for therapy, they can be incorporated into gene therapy vectors such as retrovirus vector, adenovirus vector, or adeno-associated virus vector.

When using these proteins or polynucleotides to treat hematopoietic diseases, the proteins or polynucleotides may be administered directly to patients. Preferably, the polynucleotides are introduced into hematopoietic stem cells, and then the hematopoietic stem cells stably carrying the introduced polynucleotides are selected and transplanted into patients. When introducing the polynucleotides into hematopoietic stem cells, the polynucleotides may be inserted into gene therapy vectors, as described above.

When treating hematopoietic diseases using these proteins or polynucleotides, the proteins or polynucleotides may be used as they are, or they may be formulated into pharmaceutical formulations by known pharmaceutical methods.

When preparing pharmaceutical formulations, the effective ingredients, such as the above-mentioned polynucleotides and proteins, may be combined with other suitable solutes and solvents. For example, the compound can be administered by formulating in combination with pharmacologically acceptable carriers or vehicles such as sterile water, physiological saline, vegetable oils, emulsifying agents, and suspending agents, as appropriate. The pharmaceutical compositions of the present invention can be in any form such as aqueous solutions, tablets, capsules, troches, buccal tablets, elixirs, suspensions, syrups, nasal drops, and inhalants. Content of the compound may be determined as appropriate. Administration to a patient can be generally conducted according to techniques known by one skilled in the art, for example, intraarterial injection, intravenous injection, hypodermic injection, oral administration, and intraarticular injection.

The dosage varies with the body weight and age of the patient, administration method, symptoms, and such, but one skilled in the art can appropriately choose a suitable dosage. While a general dosage varies depending on the effective blood concentration and metabolic rate of an agent, the daily maintenance dose is considered to be about 0.1 mg/kg to about 1.0g/kg, preferably about 0.1 mg/kg to about 10 mg/kg, and more preferably about 0.1 mg/kg to about 1.0 mg/kg. The administration can be conducted in one to several times. If the compound can be encoded by a polynucleotide, gene therapy can be conducted by incorporating the polynucleotide into a gene therapy vector.

Hematopoietic stem cells introduced with a polynucleotide encoding synoviolin or a protein functionally equivalent to synoviolin may be used as a pharmaceutical formulation. To obtain hematopoietic cells, multipotent human stem cells must be isolated from the bone marrow, or from other hematopoietic sources. First, bone marrow cells can be obtained from bone marrow sources such as the iliac crest, tibia, femur, spine, or other bone cavities. Other sources for obtaining hematopoietic stem cells include embryonic yolk sac, fetal liver, fetal and adult spleen, adult peripheral blood, and umbilical cord blood.

Hematopoietic stem cells can be obtained from these tissues by following the procedure of Herenberg, L. A. "Weir's Handbook of Experimental Immunology, 5th edition," Blackwell Science Inc. 1997. More specifically, cells can be immunologically stained using anti-CD34 antibodies, anti-CD33 antibodies, anti-CD38 antibodies, and such, and then sorted based on the stainability of these antibodies using a cell sorter.

After hematopoietic stem cells are isolated, they can be grown as follows. Specifically, hematopoietic stem cells can be grown by providing growth factors that are involved in stem cell maintenance by co-culturing these cells with stromal cells obtained from bone marrow cells, fetal thymus, or fetal liver.

For introduction of therapeutic genes into hematopoietic stem cells, or hematopoietic precursor cells, methods generally used for gene introduction into animal cells may be used, including methods that use virus-derived gene therapy vectors for animal cells, such as retrovirus vectors, adenovirus vectors, adeno-associated virus (AAV) vectors, simple herpes virus vectors, and HIV vectors, the calcium phosphate co-precipitation method, DEAE-dextran method, electroporation method, liposome method, lipofection method, and microinjection method.

The pharmaceutical methods or pharmaceutical formulations described above supplement patient's body with synoviolin. Accordingly, the ER stress-induced apoptosis is inhibited, and abnormal erythroblast differentiation and such are overcome or reduced. As a result, treatments for diseases caused by abnormal hematopoietic processes such as abnormal erythroblast differentiation are expected.

The present invention also relates to methods for treating hematopoietic diseases and to therapeutic formulations for hematopoietic diseases which utilize substances that may enhance the activity of synoviolin. The present inventors isolated a naturally-occurring synoviolin ligand (SL). SL and a known gene called S1-5 (Lecka-Czernik, B. *et al.,* Molecular and Cellular Biology, 15, 120-128, 1995) have nearly the same partial DNA sequences. In addition, their genetic size and the molecular weight of their expression products are approximately the same; therefore, they are very likely to be the same protein. Other SLs that may be used are accession numbers AAA65590 (nucleotide accession U03877), I38449, NP_061489 (nucleotide accession NM_018894), NP_004096 (nucleotide accession NM_004105), and similar proteins that have the activity to bind human synoviolin protein (SEQ ID NO: 2) (Lecka-Czernik, B. *et al.,* Mol. Cell. Biol. 15, 120-128, 1995; Heon, E. *et al.,* Arch. Ophthalmol. 114, 193-198, 199.6; Ikegawa, S. *et al.,* Genomics 35, 590-592, 1996; Katsanis, N. *et al.,* Hum. Genet. 106, 66-72, 2000; Giltay, R. *et al.,* Matrix Biol. 18, 469-480, 1999; Stone, E. M. *et al.,* Nat. Genet. 22, 199-202, 1999).

Synoviolin activity-enhancing agents that can stimulate synoviolin activity, such as these synoviolin ligands or polynucleotides encoding them, may be utilized in the methods for treating hematopoietic diseases caused by decreased synoviolin function.

When such synoviolin ligands are used in pharmaceutical formulations, SL proteins and polynucleotides encoding these proteins can be combined with other suitable solutes and solvents in addition to the effective ingredients as described above. Hematopoietic cells introduced with polynucleotides encoding SLs may also be used as pharmaceutical formulations.

The present invention increases the expression or activity of synoviolin by administering synoviolin ligands. Accordingly, the present invention is expected to treat hematopoietic diseases involving decrease of synoviolin expression or activity.

The present invention relates to methods for inducing erythroblast differentiation using synoviolin. As described above, abnormal erythroblast differentiation has been observed in embryos whose synoviolin gene is homozygously deleted. Therefore, synoviolin, synoviolin-functionally equivalent proteins, and polynucleotides encoding these proteins may be utilized for reagents and methods for inducing erythroblast differentiation.

Methods for inducing erythroblast differentiation are carried out by administering the above-mentioned synoviolin or a synoviolin-functionally equivalent protein to hematopoietic stem cells, or by expressing the protein in cells. A polynucleotide encoding synoviolin or a synoviolin-functionally equivalent protein is inserted into an expression vector, and this vector is introduced into hematopoietic stem cells. Isolation of hematopoietic stem cells, types of expression vectors that may be used, and methods of introduction are as described above. After introducing a polynucleotide encoding synoviolin or such, the cells are cultured, the morphology is examined, and the cells that differentiated into erythroblasts are collected. Synoviolin may also be used in combination with other substances that can induce erythroblast differentiation. For example, differentiation to CFU-E is known to take place when erythropoietin alone is used as the inducer for erythroblast differentiation, while differentiation to BFU-E is known to take place when SCF is used in combination with erythropoietin. Therefore, by using an inducer in combination with other inducers, cell differentiation may be induced differently from when the inducer is used alone.

As described above, polynucleotides encoding synoviolin or synoviolin-functionally equivalent proteins, or expression vectors carrying such polynucleotides may have applications as reagents, such as erythroblast differentiation inducers.

The present invention relates to non-human hematopoietic disease model animal embryos whose synoviolin gene function is defective. The present inventors attempted to produce knockout mice for functional analysis of synoviolin; however, embryos whose synoviolin was homozygously deleted died at approximately E13.5. Analysis of the embryos before death revealed augmented apoptosis, abnormal erythroblast differentiation, and macrophage activation induced by these abnormal cells and therefore hemophagocytosis. Thus, synoviolin homozygous knockout embryos may become model embryos for hematopoietic diseases, in particular, diseases caused by abnormal erythroblast differentiation. This abnormal erythroblast differentiation is presumed to be caused mainly by ER stress-induced apoptosis, therefore, synoviolin homozygous knockout embryos may become models for diseases caused by ER stress-induced apoptosis.

The non-human animal models of the present invention are preferably mammals (such as mice, rats, hamsters, rabbits, pigs, goats, sheep, horses, and cattle), and in particular, the use of rodents such as mice or rats is suitable.

Non-human animals with defective synoviolin gene function can be produced by methods such as gene targeting. For example, to produce such non-human animal models, a targeting vector in which a portion or all of the DNA of the present invention is made defective by substitution, deletion, addition, insertion, and/or such is inserted into embryonic stem (ES) cells, and cells that have undergone homologous recombination with chromosomal DNA are selected. Conventional positive/negative selection can be carried out to select homologous recombinants. Examples of markers used for positive selection include drug resistance genes such as neomycin resistance gene, and examples of markers for negative selection include diphtheriatoxin (DT)-A gene and HSV-tk gene. Correctly recombined cells can be selected by Southern blotting, PCR, and such. The cells thus obtained are injected into fertilized eggs at roughly the 8-cell stage, or into the blastocoel of a blastocyst, and then these cells are transferred to the uterus of a pseudopregnant female individual prepared by mating with a vasectomized male. Genomic DNA analysis of the littermates can be carried out based on the Examples described below. Samples such as blood or tissues can be obtained from the littermates, and DNA analysis can be performed based on the obtained samples. Whether the individual is a heterozygous knockout mouse can be determined from this DNA analysis.

To obtain homozygous knockout mice, heterozygous knockout mice are crossed with each other. DNA analysis of the embryos obtained as a result of the crossing is performed as described above to identify homozygous knockout mice. In the case of mice, the homozygous knockout dies at around E13.5. Therefore, to obtain living mouse embryos, they are removed from the uterus before E 13.5.

Animals with defective synoviolin function can be generated not only by knocking out the synoviolin gene, but by combining this gene knock out step with a step of knocking in another gene. The gene to be knocked in is not particularly limited, and examples include marker genes such as lacZ gene.

In addition to gene targeting as a means for deleting synoviolin function, inhibiting the post-transcription expression without modifying the synoviolin gene on chromosomal DNA, such as the antisense method, ribozyme method, or iRNA method may be used as the means to turn off synoviolin function. In the antisense method, a vector comprising a DNA encoding an RNA complementary to the transcription product of a DNA encoding the protein of the present invention is used. In the ribozyme method, for example, a vector comprising a DNA encoding an RNA that cleaves the transcription product of a DNA encoding the protein of the present invention is used. The vector is introduced into mammalian embryonic stem cells as described above, and these cells are injected into mammalian embryos, from which individuals can then be obtained.

In the iRNA method, a vector for expressing a DNA encoding the synoviolin protein, for example, a double stranded RNA is introduced into mammalian embryonic stem cells as described above, wherein the antisense RNA complementary to a transcription product comprising the sequence of SEQ ID NO: 1 is paired with a sense RNA complementary to this antisense RNA, and these cells are inserted into mammalian embryos, from which individuals can then be obtained.

As described above, embryos with defective synoviolin function develop a property of abnormal erythroblast differentiation. Such property is expressed from E10.5 and thereafter. Therefore, embryos that are E10.5 or older but E13.5 or younger and are still alive will become models of hematopoietic diseases, in particular, diseases caused by abnormal erythroblast differentiation. In addition, not only the embryos themselves but cells derived from the embryos may also be utilized as model cells. Suitable examples of embryo-derived cells are MEFs and such. These model embryonic animals and cells derived from them can be used to test or screen for various compounds including candidate pharmaceutical compounds that act against hematopoietic diseases, and in particular diseases caused by abnormal erythroblast differentiation. Examples of methods that utilize the model fetal animals of the present invention for testing or screening include the following methods.

In the present invention, the methods of screening for therapeutic agents for hematopoietic diseases involve administering test substances to non-human hematopoietic disease model animal embryos whose synoviolin gene function is defective, or their mothers, and evaluating the condition of erythroblasts in the non-human animal embryos. When erythroblasts differentiate normally in the same degree as the wild type, or when the value indicating abnormal differentiation is decreased compared to that for homozygous knockout embryos, the test substance becomes a candidate for therapeutic agents for hematopoietic diseases.

In another embodiment of the screening method, a screening method comprising the following steps (a) to (c) is shown:
(a) administering or contacting an ER stress-inducing agent to non-human hematopoietic disease model animal embryos whose synoviolin gene function is defective, or cells derived from the embryos;
(b) administering or contacting a test substance with the nonhuman hematopoietic disease model animal embryos whose synoviolin gene function is defective, or cells derived from the embryos, before, after, or simultaneously with step (a); and
(c) identifying cells whose apoptosis was induced among the non-human animal embryos or the embryo-derived cells.

The above-mentioned model embryos whose synoviolin function has been deleted may be used for the non-human hematopoietic disease model animal embryos in the screening method. For the embryo-derived cells, cells collected from the model embryos, or MEFs may be suitably used.

ER stress agents that are made to act on these model embryos or cells derived from the embryos such as tunicamycin, thapsigargin, and other pharmaceutical agents which can induce ER stress, may be used. The embryos or cells derived from the embryos are subjected to ER stress by these ER stress agents.

Stress induction by ER stress agents may be carried out by direct administration to embryos, or by administration into cultures of growing embryos. Stress in embryo-derived cells may be induced by adding ER stress agents into cultures of growing cells.

Test substances are administered to or contacted with the model embryos or cells derived from the embryos before, after, or simultaneously with the above-mentioned ER stress induction step. This method of administrating and contacting test substances can be appropriately selected according to the type of test substance and such. Administration to embryos can be carried out, for example, by addition into cultures of growing embryos, or by direct injection into the body. For cells, the test compounds can be added into the media. Injection into cells by microinjection or such is also possible. When the test substance is a gene, the gene can be introduced into cells in combination with the desired transfection reagents as naked DNA, or by incorporation into a conventional expression vector.

After effecting the test substance, non-human animal embryos or embryo-derived cells were assayed for cells whose apoptosis was induced. The assay for apoptotic cells can be carried out by TUNEL assay indicated in the Examples. TUNEL assay may be carried out using commercially available kits. After assaying for apoptotic cells, the number of apoptotic cells in groups where a test substance was effected was compared to that in groups without such effect. If a significant decrease in the number of apoptotic cells is observed in groups where a test substance was effected, the test substance is judged to have an activity of suppressing ER stress induction, and may induce normal differentiation of erythroblasts.

Although there are no particular limitations on the test compound used for the screening, examples include inorganic compounds, organic compounds, peptides, proteins, natural or synthetic low molecular weight compounds, natural or synthetic high molecular weight compounds, tissues or cell extracts, culture supernatants of microorganisms, and natural components derived from plants and marine organisms, but it is not to be construed as being limited thereto. Expression products of gene libraries, expression cDNA libraries, or such may be used.

All prior art literature cited herein are incorporated into this description as references.

### Brief Description of the Drawings

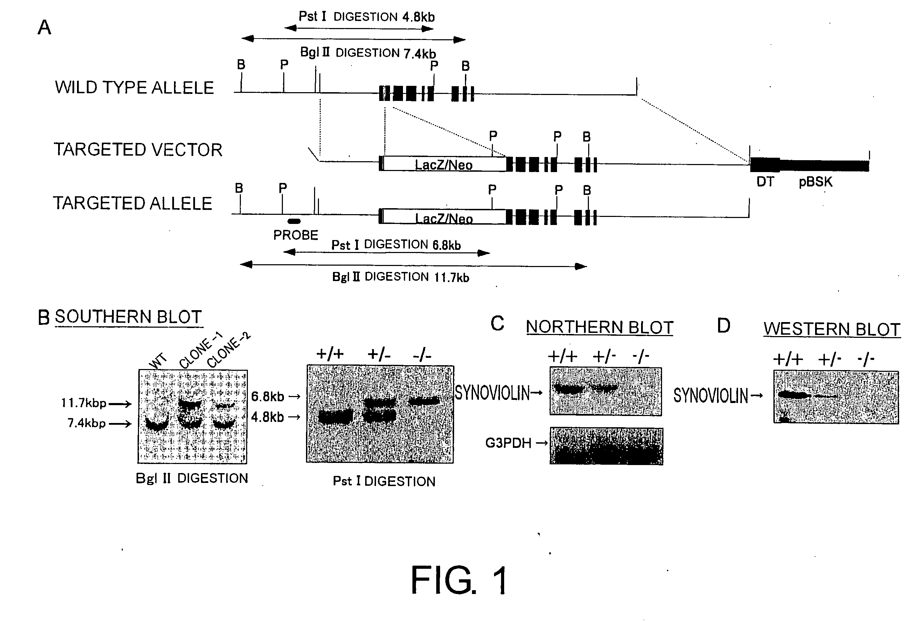

Fig. 1 is a set of diagrams and photographs showing targeted disruption of the synoviolin gene. Fig. 1(A) shows the structure of the synoviolin wild-type allele, the targeting vector, targeted allele, and partial restriction enzyme map of the genes before and after targeting events. Exons of the gene are shown as closed boxes, and the β-galactosidase gene (LacZ), neomycin phosphotransferase gene (Neo), diphtheria toxin-A gene (DT), and pBluescript II (BSK) are shown as open boxes. The restriction sites used are indicated as: B for *Bgl*II*;* P for *Pst*I*;* E for *EcoR*I*;* X for *Xho*I; and N for *Nco*I. Fig. 1(B) is a set of photographs showing the Southern blot analysis of targeted ES clones and embryonic clones generated by crossing heterozygous knockout (hereinafter denoted as "syno^{+/-}") mice with each other. Genomic DNA from wild-type (hereinafter denoted as "syno^{+/+}") TT2 ES cells (WT) and homologously targeted ES clones (clone-1, clone-2) were digested with *Bgl*II and hybridized with an external probe. The syno^{+/+} and syno^{+/-} alleles produced 7.4 kb and 11.7 kb fragments, respectively. Chromosomal DNA isolated from E13.0 embryos generated by crossing syno^{+/-} mice was digested with *Pst*I, and hybridized using the same probe as described above. Fig. 1 (C) is a photograph of Northern blot analysis. Twenty µg of total RNA, isolated from E13.0 embryos generated by crossing *syno*^{+/-} mice, was hybridized with a synoviolin probe or glyceraldehyde-3-phosphate dehydrogenase (G3PDH) probe. Fig. 1(D) is a photograph showing the result of isolating proteins from E13.0 embryos and separating them by SES-PAGE (50 µg protein/lane). The proteins were transferred to a membrane, and then reacted with an anti-synoviolin antibody.

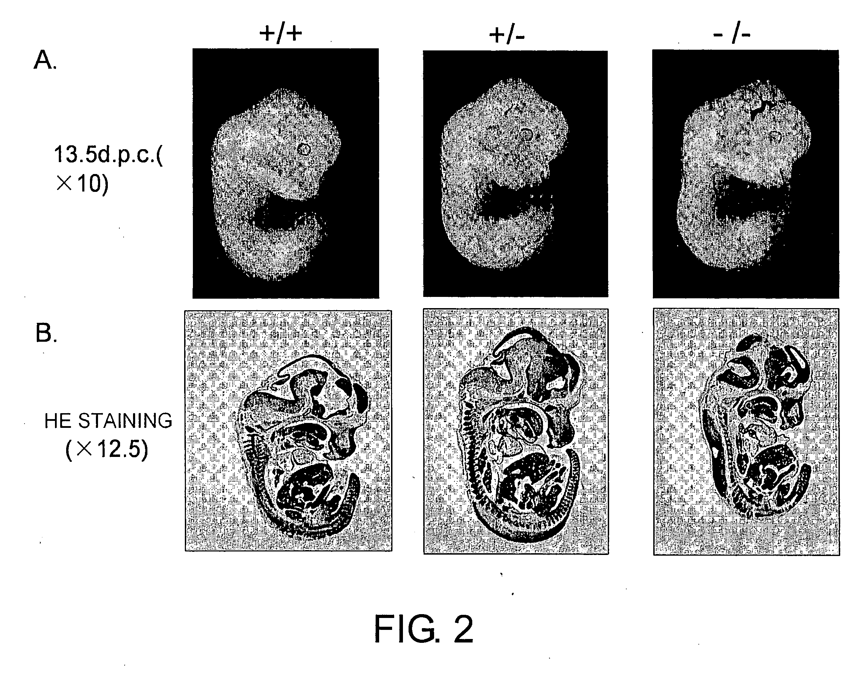

Fig. 2 is a set of photographs indicating the phenotypes of E13.5 homozygous knockout (hereinafter denoted as "syno^{-/-}") embryos. Fig. 2(A) shows the external appearance of syno^{+/+}, syno^{+/-}, and syno^{-/-} E13.5 mouse embryos. The mutant embryos has an equal size as syno^{+/+} and show normal development. Fig. 2(B) is a set of photographs showing the tissue sections of syno^{+/+}, syno^{+/-}, and syno^{-/-} E13.5 mouse embryos stained with hematoxylin and eosin (x 10).

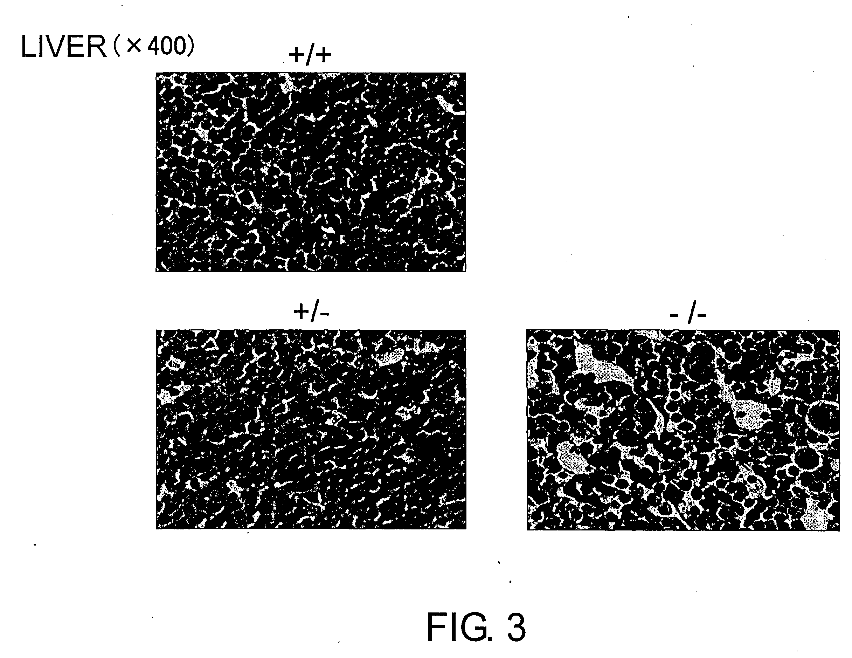

Fig. 3 is a set of photographs showing the histology of liver from the E13.5 embryos. Details of the liver sections of syno^{+/+}, syno^{+/-}, and syno^{-/-} E13.5 are shown (x 400). Cell density was found to be low in the syno^{-/-} embryo.

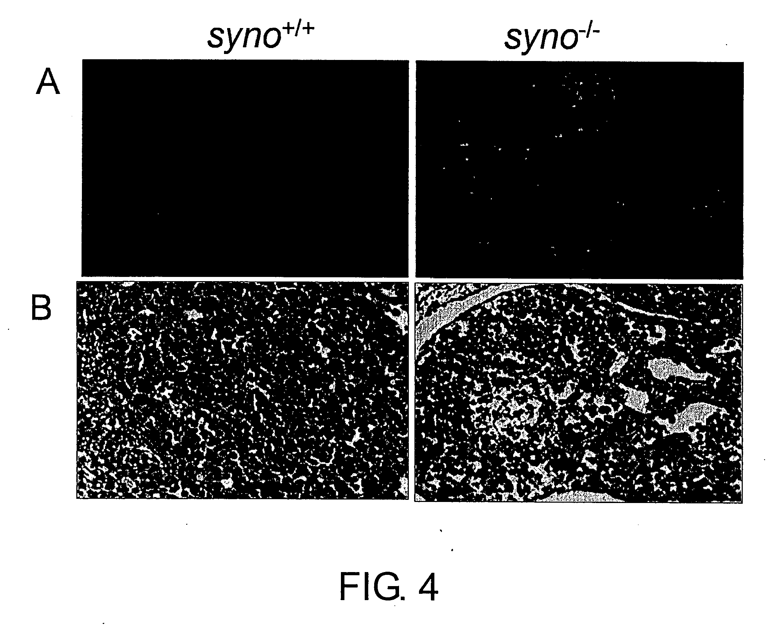

Fig. 4 is a set of photographs indicating the result of analyzing the proportion of apoptotic cells in the syno^{-/-} embryo-derived liver cells. (A) shows the result of TUNEL assay on liver tissues derived from the syno^{+/+} and syno^{-/-} embryos (x 200). TUNEL positivity of syno^{+/+} was 1%, whereas that of syno^{-/-} was a high 25%. This difference which was observed in the liver was also observed throughout the entire syno^{-/-} embryos. (B) Hematoxylin and eosin staining of the syno^{+/+} and syno^{-/-} embryos are shown (x 200).

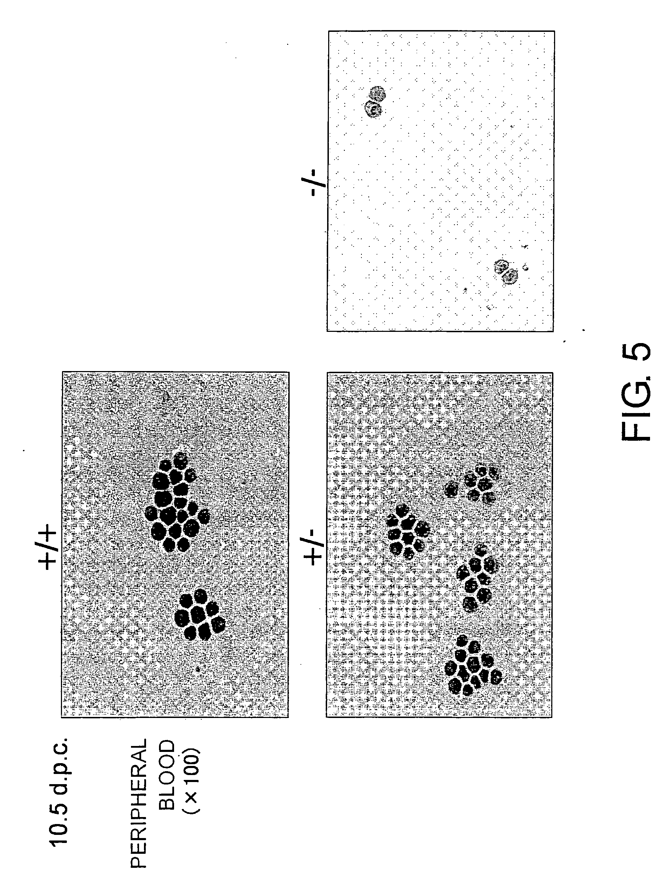

Fig. 5 is a set of photographs showing hematopoiesis in E10.5 syno^{-/-} embryos. Cytocentrifuge preparations of peripheral blood isolated from E10.5 syno^{+/+}, syno^{+/-}, and syno^{-/-} embryos stained with May-Grünwald-Giemsa are shown. Syno^{-/-} embryos showed reduced erythroblast formation and augmented apoptosis (indicated by arrows).

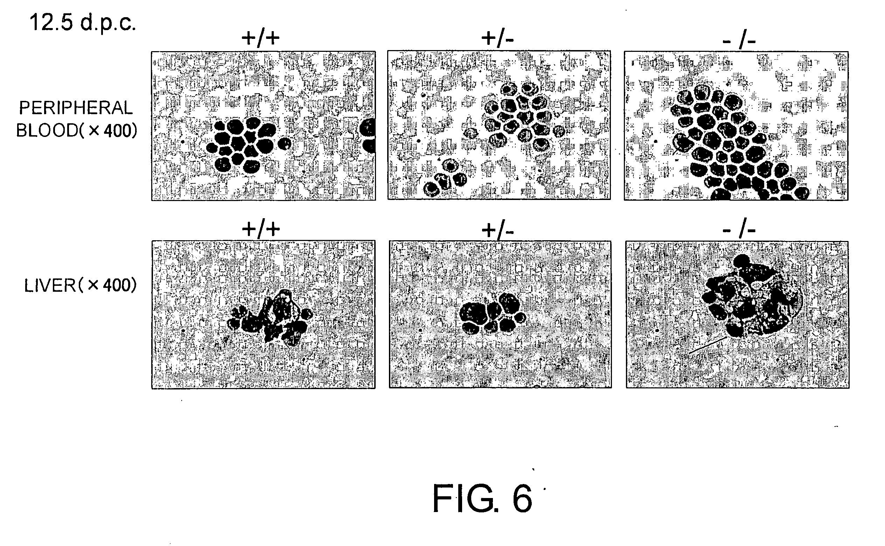

Fig. 6 is a set of photographs showing hematopoiesis in E 12.5 syno^{-/-} embryos. The upper row shows the result of analyzing the cytocentrifuge preparations of peripheral blood isolated from viable E12.5 embryos. Abnormal erythroblasts, nuclear fragmentation and Howell-Jolly bodies are increased in the syno -/- embryos compared to the syno^{+/+} embryos (indicated by an arrow). The lower row shows the result of analyzing liver cytocentrifuge preparations from the May-Grünwald-Giemsa-stained E12.5 syno^{+/+} and syno -/- embryos. Hemophagocytosis is clearly increased in the syno^{-/-} embryos (indicated by an arrow).

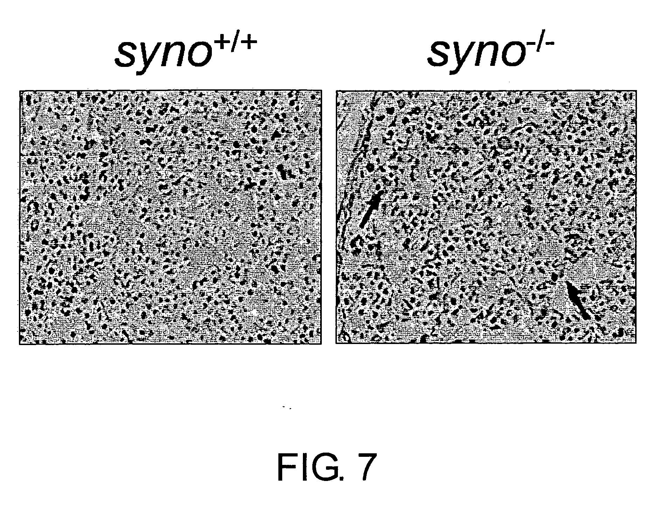

Fig. 7 is a set of photographs showing the result of analyzing the β-globin expression in livers derived from E12.5 syno^{+/+} and homozygous knockout syno^{-/-} embryos by immunohistological staining (x 200). β-globin expression is decreased in the syno^{-/-} embryos compared to syno^{+/+} (syno^{+/+}: 4.60%, syno^{-/-}: 0.90%, indicated by arrows).

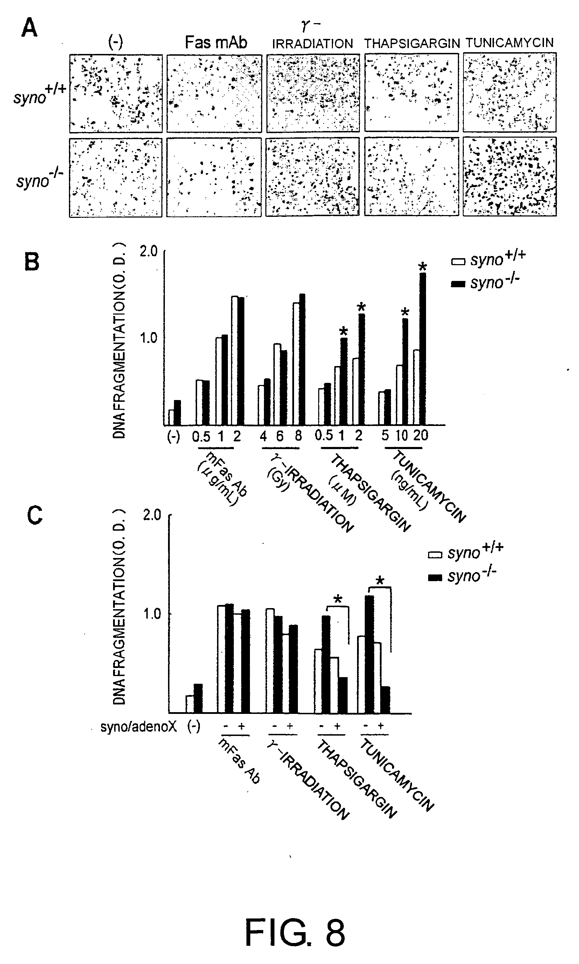

Fig. 8 is a set of photographs and graphs showing increased susceptibility to ER stress in syno^{-/-} MEFs. (A) is a set of photographs showing the result of treatment with various apoptotic stimuli, including anti-Fas monoclonal antibodies (1 µg/ml for 48 hours), X-irradiation (6 Gy for 72 hours), thapsigargin (1 µM for 48 hours) and tunicamycin (10 ng/ml for 48 hours), or untreatment (-) on MEFs derived from syno^{+/+} and syno^{-/-} mice, in low serum (1% FCS) medium. Apoptotic cells were detected by TUNEL analysis. (B) is a graph indicating the result of treatment with indicated doses of stimuli on syno^{+/+} and syno^{-/-} MEFs. Apoptosis was measured by quantitation of DNA fragmentation using the cell death detection ELISA method (Boehringer Mannheim). *p<0.01. The number of apoptotic syno^{-/-} MEFs was higher in cultures treated with thapsigargin and tunicamycin compared with the respective syno^{+/+} MEFs. (C) is a graph indicating the result of infection with an adenovirus vector (100 moi) carrying LacZ(-) or synoviolin gene(+) on syno^{+/+} and syno^{-/-} MEFs, followed by treatment with the same aforementioned agents. In the syno^{-/-} MEFs, ER stress-induced apoptosis was rescued by synoviolin expression.

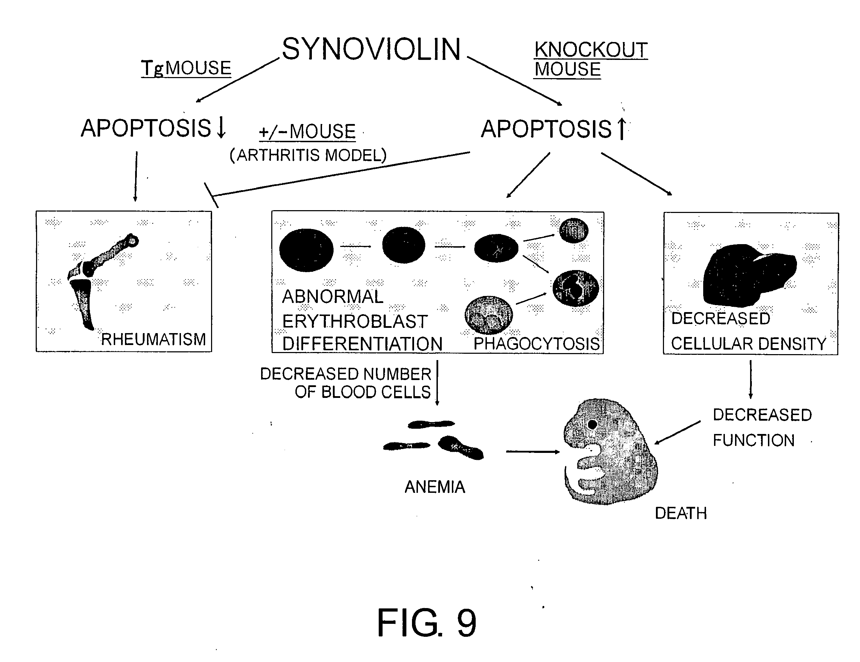

Fig. 9. is a diagram indicating that synoviolin acts as an anti-apoptotic protein through ERAD system. Synovioin's 'loss-of-function' results in generalized apoptosis through breakdown of the ERAD system. This process results in decreased cellular density and abnormal hematopoiesis, and consequently causes embryonic death. On the other hand, synoviolin's 'gain-of function' causes the onset of spontaneous arthropathy through its anti-apoptotic effects. Furthermore, syno^{+/-} mice are resistant to collagen-induced arthritis (CIA), which is a model frequently used in experiments related to arthritis. Taken together, synoviolin plays an important role in the 'maintenance of cellular function' through ERAD system.

### Best Mode for Carrying Out the Invention

The present invention is illustrated in detail below by the Examples, but is not to be construed as being limited thereto.

### [Example 1] Generation of synoviolin gene knockout mice

To disrupt the mouse synoviolin gene, the present inventors constructed a targeting vector (Fig. 1). The synoviolin cDNA was cloned from C57BL/6 genomic library for vector construction. An *Nco*I site was created at the translation initiation codon of the gene and then the *Nco*I/*BamH*I fragment of the gene was replaced with the LacZ cassette (Saga, Y., Yagi, T., Ikawa, Y., Sakakura, T., and Aizawa, S., 1992, "Mice develop normally without tenascin." Genes Dev. 6: 1821-1831). The neomycin phosphotransferase (Neo) gene cassette derived from pMClneo (Strategene, La Jolla, CA) was placed downstream of the LacZ gene. The 1.85-kb *ECOR*I/*Nco*I fragment and the 8.5-kb *Sal*I/*Xho*I fragment of the synoviolin gene were included upstream and downstream of these cassettes, respectively. Negative selection with the DT-A cassette has been described previously (Yagi, T., Nada, S., Watanabe, N., Tamemoto, H., Kohmura, N., Ikawa, Y, and Aizawa, S., 1993, "A novel negative selection for homologous recombinants using diphtheria toxin A fragment gene." Anal. Biochem. 214: 77-86).

The TT2 ES cells, derived from an F1 embryo resulted from a cross between C57BL/6 and CBA mice, were grown on feeder cells as previously described (Yagi, T., Tokunaga, T., Furuta, Y., Nada, S., Yoshida, M., Tsukada, T., Saga, Y., Takeda, N., Ikawa, Y., and Aizawa, S., 1993, "A novel ES cell line, TT2, with high germline-differentiating potency." Anal. Biochem. 214: 70-76). The targeting vector was introduced into these TT2 ES cells by electroporation. After vector introduction, if homologous recombination between this vector and the endogenous synoviolin locus occurs, an in-frame lacZ reporter gene and a neomycin resistance gene are inserted at the ATG translational start site in the synoviolin locus. Since the lacZ cassette contains a poly(A) addition sequence, it is expected to prevent the translation of synoviolin fusion protein.

After electroporation, neo-resistant TT2 ES clones were selected using agent G418 and isolated. Chromosomal DNAs were collected from the isolated clones and digested with restriction enzyme *Bgl*II*.* The restriction enzyme digests were separated by electrophoresis, and then Southern blot analysis using the probe shown in Fig. 1A was carried out for genotyping. The homologous recombinants were confirmed by Southern blot analysis (Fig. 1 B left).

Two independent syno^{+/-} ES clones were injected into ICR 8-cell stage embryos (Yagi, T., Nada, S., Watanabe, N., Tamemoto, H., Kohmura, N., Ikawa, Y, and Aizawa, S., 1993, "A novel negative selection for homologous recombinants using diphtheria toxin A fragment gene." Anal. Biochem. 214: 77-86). Chimeric males with greater than 80% agouti coat color were crossed with C57BL/6 or DBA1 females, and germ line transmission of the mutant allele was identified by Southern blot analysis or by PCR analysis of tail- and yolk sac-derived genomic DNAs (Fig. 1B, left). The following primers were used for genotyping of the embryos; 5'-ACACAGTCACCTCCGGTTCTGTATTCACTG-3' (the region indicated by P1 in Fig. 1A) and
5'-CTCAGTAACAGCGTACCAGGACCGTTCCAG-3'.

PCR was performed using these primers. PCR was carried out in 35 cycles of 96°C, 2 minutes, 96°C, 30 seconds, 60°C, 30 seconds, and 72°C, 3 minutes, followed by an extension reaction of 10 minutes at 72°C after the last cycle, and subsequent cooling at 4°C. This PCR analysis produces 6.9 kb and 2.6 kb fragments from the mutant and wild type alleles, respectively.

Two independently targeted clones described above were injected into ICR 8-cell stage embryos and gave germ line transmission (Fig. 1B, left). Heterozygous syno^{+/-} F1 mice for the mutation were viable, fertile, and had no apparent phenotypic abnormalities (data not shown). syno^{+/-} mice were interbred to generate syno^{-/-} mice, and newborn offspring were genotyped, but no syno^{-/-} mice were identified. Therefore, the possibility that synoviolin deficiency causes embryonic death was considered. Analysis of the embryos at various developmental stages clearly showed that they died by approximately E 13.5 (Table 1).

**[Table 1]**

| Stage | Total (number of littermate group) | Synoviolin | | | |
|---|---|---|---|---|---|
| | | +/+ | +/- | -/- (alive) | -/- (dead) |
| Postnatal | 10 (2) | 2 | 8 | 0 | |
| E18.5 | 3(1) | 3 | 0 | 0 | |
| E17.5 | 4(1) | 0 | 4 | 0 | |
| E15.5 | 5(1) | 1 | 4 | 0 | |
| E14.5 | 50 (8) | 22 | 22 | 0 | 6 |
| E13.5 | 81(13) | 27 | 47 | 2 | 5 |
| E12.5 | 104(17) | 26 | 67 | 10 | 1 |
| E11.5 | 35 (5) | 8 | 18 | 8 | 1 |
| E10.5 | 26(4) | 8 | 12 | 6 | 0 |
| Total | 318(51) | 97 | 182 | 39 | |

To determine the nature of this targeted synoviolin mutation, genomic DNA, total RNA, and protein were prepared from each of the E13.0 syno^{+/+}, syno^{+/-}, and syno^{-/-} embryos, and analyzed by Southern, Northern, and Western blottings.

Total RNA was isolated from E13.0 embryos using Isogen (Nippon Gene) based on the acid guanidinium thiocyanate phenol chloroform extraction method (Chomczynski, P., and Sacchi, N., 1987, "Single-step method of RNA isolation by acid guanidinium thiocyanate-phenol-chloroform extraction." Anal. Biochem. 162: 156-159). Then, 20 µg of total RNA were denatured with glyoxal, separated by electrophoresis, and transferred onto a nylon membrane. The membrane was hybridized with a DNA probe corresponding to bases 1235-3028 of the cloned synoviolin cDNA fragment.

As shown in Fig. 1B right, 1C and 1D, synoviolin gene was deficient and no synoviolin mRNA or protein was detected in the syno^{-/-} embryos. Thus, the present inventors confirmed that the syno^{-/-} embryos expressed neither synoviolin transcript nor synoviolin protein.

### [Example 2] Apoptotic cell death in syno^{-/-} embryos

To identify the stage of embryonic development at which the synoviolin mutation is lethal, the present inventors analyzed E10.5-E18.5 embryos. For the analysis, embryos were removed from the uterus. Yolk sac was collected for genotyping. For histological analysis of the embryos, the embryos were fixed overnight in 4% paraformaldehyde in phosphate-buffered saline (PBS) and embedded in paraffin, and 4-µm sections were incised and stained with hematoxylin and eosin, and some sections were used for the TUNEL assay. Peripheral blood was collected in PBS containing 50% FCS and in 10 mM EDTA for cytocentrifuge preparation. Fetal livers from E12.5 embryos were disrupted in 1 mL of the same medium with a 25-gauge needle, and 7 µL was diluted into 200 µL of medium and cytocentrifuged. Slides of peripheral blood and fetal liver were stained with May-Grünwald-Giemsa.

At E11.5, the majority of the syno^{-/-} embryos (88.9%) were viable; however, by E13.5, very few syno^{-/-} embryos were found to be alive (Table 1). Morphological analysis of the embryos at E13.5 revealed no obvious difference in external appearance among the syno^{-/-}, syno^{+/-}, and syno^{+/+} littermates (Fig. 2A). Histological examination also did not reveal a serious difference in the syno^{-/-} embryos, such as absence of major organs (Fig. 2B).

However, histological examination of the syno^{-/-} embryo livers in narrow image revealed that the cellular density was decreased throughout the entire syno^{-/-} embryos compared to the syno^{+/+} and syno^{+/-} embryos (Fig. 3). We assumed that the low cell density was due to augmented apoptotic cell death in the syno^{-/-} embryos.

To determine the extent of apoptotic cell death induced by the loss of synoviolin, TUNEL analysis was performed at each stage of the embryo. Specifically, liver tissues and sections were prepared as described above. These specimens were subjected to TUNEL analysis. TUNEL analysis was performed according to the protocol provided by the manufacturer (MEBSTAIN Apoptosis kit II, Medical & Biological Laboratories, Nagoya, Japan). In three separate areas, the number of TUNEL-positive cells among 1,000 cells was counted, and the rate of TUNEL-positive cells was calculated.

At E.12.5, apoptotic cell death was clearly detected, notably in the liver. The number of apoptotic cells was significantly increased in the syno^{-/-} embryos (25%) compared to the syno^{+/+}embryos (1%) (Fig. 4A). Embryos at this stage express synoviolin only in particular sites such as the hematopoietic cells (data not shown).

### [Example 3] Aberrant apoptosis in hematopoiesis of syno^{-/-} embryos

In the normal development process of murine embryos, the major organ of hematopoiesis shifts at around E11.5-E12.5 from the yolk sac to fetal liver (Zon, L.I., 1995, "Developmental biology of hematopoiesis." Blood 86: 2876-2891). Based on analyses of the various genes in a null mutant mouse, several studies have concluded that impairment of hematopoiesis is often associated lethality at this stage (Nuez, B., Michalovich, D., Bygrave, A., Ploemacher, R., and Grosveld, F., 1995, "Defective haematopoiesis in fetal liver resulting from inactivation of the EKLF gene." Nature 375: 316-318; Okuda, T., van Deursen, J., Hiebert, S.W., Grosveld, G, and Downing, J.R., 1996, "AML1, the target of multiple chromosomal translocations in human leukemia, is essential for normal fetal liver hematopoiesis." Cell 84: 321-330; Kieran, M.W., Perkins, A.C., Orkin, S.H., and Zon, L.I., 1996, "Thrombopoietin rescues *in vitro* erythroid colony formation from mouse embryos lacking the erythropoietin receptor." Proc. Natl. Acad. Sci. U.S.A. 93: 9126-9131; Wang, Q., Stacy, T., Miller, J.D., Lewis, A.F., Gu, T.L., Huang, X., Bushweller, J.H., Bories, J.C., Alt, F.W., Ryan, G., Liu, P.P., Wynshaw-Boris, A., Binder, M., Marin-Padilla, M., Sharpe, A.H., and Speck, N.A., 1996, "The CBFbeta subunit is essential for CBFalpha2 (AML1) function *in vivo*." Cell 87: 697-708; Tamura, K., Sudo, T., Senftleben, U., Dadak, A.M., Johnson, R., and Karin, M., 2000, "Requirement for p38alpha in erythropoietin expression: a role for stress kinases in erythropoiesis." Cell 102: 221-231; Spyropoulos, D.D., Pharr, P.N., Lavenburg, K.R., Jackers, P., Papas, T.S., Ogawa, M., and Watson, D.K., 2000, "Hemorrhage, impaired hematopoiesis, and lethality in mouse embryos carrying a targeted disruption of the Fli1 transcription factor." Mol. Cell Biol. 20: 5643-5652; Kawane, K., Fukuyama, H., Kondoh, G., Takeda, J., Ohsawa, Y, Uchiyama, Y., and Nagata, S., 2001, "Requirement of DNase II for definitive erythropoiesis in the mouse fetal liver." Science 292: 1546-1549). To investigate the status of hematopoiesis in syno^{-/-} embryos, cytocentrifuge preparations of E10.5 and E12.5 peripheral blood, and E12.5 fetal liver were examined.

To analyze the status of primary hematopoiesis, peripheral blood samples obtained from E10.5 were first stained and then compared with the erythroblasts formation in syno^{+/+} and syno^{+/-} littermates (3.0±0.66x 10⁵ cells) (Fig. 5). The syno^{-/-} embryos (8.3±0.46x 10⁵ cells) showed diminished erythroblasts formation compared with the syno^{+/+} and syno^{+/-} embryos. Moreover, apoptosis was observed in syno^{-/-} erythrocytes, but not in syno^{+/+} erythroblasts (data not shown).

Next, peripheral blood of an E12.5 embryo undergoing secondary hematopoiesis was Giemsa stained. Herein, erythroblasts in a further advanced stage of differentiation compared to the previously mentioned erythroblasts formed by primary hematopoiesis were observed, and mature enucleated erythrocytes were also observed. Staining of the E12.5 peripheral blood sample showed a decrease of erythroblasts in the syno^{-/-} embryos (18.0±0.19x 10⁵ cells) compared to the syno^{+/+} embryos (4.0±0.18x 10⁵ cells) (Fig. 6). In addition, the proportion of abnormal erythroblasts, such as those having Howell-Jolly bodies, those in which nuclear fragmentation has taken place, and those having two-segmented nuclei, was significantly higher in the syno^{-/-} embryos (10.5%) than in the syno^{+/+} littermates (1.8%) (Table 2, abnormal erythroblasts are indicated by an arrow in the upper row of Fig. 6).

**[Table 2]**

| Erythroblast morphology | +/+ | +/- | -/- |
|---|---|---|---|
| Nuclear disruption | 0.0% | 0.6% | 2.0% |
| Binucleation | 0.1 % | 1.2% | 2.1 % |
| Howell-Jolly body | 1.7% | 3.8% | 6.4% |

In the above observation, apoptosis of erythroblasts observed during primary hematopoiesis in the syno^{-/-} embryos was hardly observed at E12.5. To examine the reason behind this observation, the liver was stained by Giemsa. The Giemsa staining (lower row of Fig. 6) showed that images of erythroblast phagocytosis by macrophages, i.e., hemophagocytosis (lower row of Fig. 6, indicated by arrows), were hardly observed in syno^{+/+} and syno^{+/-}, whereas in syno^{-/-}, such images were significantly increased and were found in 22 out of 100 macrophages (Table 3). Therefore, erythroblasts that have undergone apoptosis similar to that observed at E10.5 were engulfed and eliminated by macrophages in the liver, which is the site of hematopoiesis, and as result, it is projected that erythroblasts that have undergone apoptosis are not found in the peripheral blood derived from E12.5.

**[Table 3]**

| Mφ phagocytosis image | +/+ | +/- | -/- |
|---|---|---|---|
| (cells/ 100Mφ) | 1 | 7 | 22 |

This phenomenon was also observed in the histological sections of syno^{-/-} fetal livers (Fig. 3). These results suggested that macrophages were activated to phagocytose abnormal erythroblasts. Under total consideration, these findings indicate that the lack of synoviolin is associated with abnormal erythroid differentiation caused by the augmentation of apoptosis, and it was likely that the syno^{-/-} embryos died of anemia as a result of the decreased number of circulating erythroblasts.

To study the involvement of synoviolin in hematopoiesis, the present inventors further examined the expression of β-globin, a secondary hematopoiesis marker, by immunohistological staining.

Immunohistological staining was performed according to a previously reported procedure (Kawahara *et al*., 1999). The sections were incubated overnight with an anti-β-globin antibody. Specific reactions between the antigen and antibody were carried out using a Diaminobenzidine Substrate Chromogen System (Vector Laboratories, Burlingame, CA) according to the attached instructions. 1000 cells in three separate regions were counted and the proportion of positive cells was calculated.

The expression of β-globin was found to be significantly decreased in syno^{-/-} compared to syno^{+/+} mice (Fig. 7). The results demonstrated that synoviolin plays an important role in hematopoiesis, especially in secondary hematopoiesis.

### [Example 4] Syno^{-/-} mouse embryonic fibroblast cells are selectively susceptible to ER stress

Apoptosis can be induced through several pathways. To identify the apoptotic pathway that describes synoviolin involvement, MEFs were isolated from syno^{-/-} and syno^{+/+} embryos. MEFs were isolated from E10.5 syno^{-/-} and syno^{+/+} embryos by trypsin digestion after removal of the heads and internal organs. Isolated cells were cultured in Dulbecco's modified Eagle's medium containing 20% fetal calf serum (FCS). MEFs were treated *in vitro* with the following four apoptotic stimuli: monoclonal anti-Fas antibodies (Abs), gamma-irradiation, tunicamycin (N-glycosylation inhibitor), and thapsigargin (Ca² -ATPase inhibitor).

Under control conditions, the proportion of syno^{-/-} MEF apoptotic cells was higher (16±4%) than syno^{+/+} MEF (6±2%) (Fig. 8A). Fas stimulation or gamma-irradiation did not alter the number of apoptotic syno^{-/-} MEFs and syno^{+/+} MEFs (Fas: syno^{-/-}, 45±2%, syno^{+/+}, 43±4%; gamma-irradiation: syno^{-/-}, 34±6%, syno^{+/+}, 31±2%, Fig. 8A). In contrast, ER stress-inducing agents, tunicamycin and thapsigargin, resulted in 1.7-fold and 2.4-fold increases in the number of TUNEL-positive syno^{-/-} MEFs, respectively, when compared with syno^{+/+} MEFs (56±3% compared with 33±7%; 90±1% compared with 38±7%, Fig. 8A). Moreover, sensitivity against the ER stress-inducing agents was increased in a dose-dependent fashion (Fig. 8B).

The present inventors investigated whether the introduction of an exogenous synoviolin gene diminishes or eliminates the effects of apoptotic stimuli on syno^{-/-} MEFs. Adenovirus vectors containing genes of Flag-tagged synoviolin or LacZ were prepared by an Adeno-X™ Expression System according to manufacturer's instructions (Clontech Laboratories, Inc., Polo Alto, CA). The viral preparations were titrated in HEK293 cells using an end-point dilution assay. The number of virus particles (measured in plaque forming units: PFU) per cell was expressed as moi. Infected MEFs were allowed to express the targeted gene for 48 hours and then treated with indicated reagents.

ER stress-induced apoptosis of syno^{-/-} MEFs was rescued by expression of synoviolin using adenovirus, although this procedure did not rescue Fas-mediated and gamma-irradiation-induced apoptosis (Fig. 8C). Under total consideration, these results indicate that synoviolin rescues MEFs from ER stress-induced apoptosis, but not from Fas- or gamma-irradiation-induced apoptosis. In addition, expressions of ER stress inducible proteins, such as CHOP/Gadd153, ATF-6, caspase-12, and such, were analyzed by Western blotting, and induction of these proteins was observed in syno^{-/-} MEFs (data not shown).

Expression of the ER stress inducible proteins including CHOP/Gadd153 and ATF-6 was induced in syno^{-/-} MEFs described above (data not shown). These proteins are considered to be involved in URP against ER stress. Therefore, in syno^{-/-} MEFs, apoptosis was thought to be induced not through the UPR system responding to ER stress, but by the breakdown of ERAD system. Namely, the ERAD system seems to be mainly involved in the quality control of proteins and it is at the same time indispensable for the 'maintenance of life,' especially during processes of embryonic development. Several ubiquitin ligases (E3) such as CHIP, gp78/AMFR, Parkin, and Fbx2/FBG1/NFB42 have been reported to be involved in the ERAD system. However, synoviolin's 'loss-of-function' causes lethality during embryonic development processes since no substitutive function is available. Our study clearly suggests that synoviolin among other ubiquitin ligases (E3) plays a pivotal role in the ERAD system, in particular.

### Industrial Applicability

To analyze the function of synoviolin, a human homologue of the yeast Hrdlp/Del3 (Bays, N.W., Gardner, R.G., Seelig, L.P., Joazeiro, C.A., and Hampton, R.Y., 2001, "Hrd1p/Der3p is a membrane-anchored ubiquitin ligase required for ER-associated degradation." Nat. Cell Biol. 3: 24-29), which is considered to play a central role in the ERAD system among ubiquitin ligases (E3), the present inventors investigated the *in vivo* function of ERAD system by generating a synoviolin-deficient mouse in the present study using embryonic stem (ES) cells.

As described above, syno^{-/-} mice died *in utero* by E12.5 to E13.5. Syno^{-/-} embryos showed apparently low cell density in several organs due to excessive apoptosis, and aberrant apoptosis was also observed in the hematopoietic system. Therefore, syno^{-/-} embryos can be utilized as models for studying or developing therapeutic agents for hematopoietic diseases, especially diseases relating to abnormal erythroblast differentiation caused by ER stress-induced apoptosis. There are other reports of knockout mice experiencing embryonic death at around E 13.5 (Table 4), but the gene which is knocked out in the models of the present invention is synoviolin, different from those reported. Therefore, the models of the present invention are expected to be useful as models of hematopoietic diseases which cannot be analyzed using models carrying defective erythropoietin (EPO) or such, and in searching for therapeutic agents against hematopoietic diseases.

**[Table 4]**

| Gene | Stage | Phenotype |
|---|---|---|
| AML1 | E12.5 | Abnormal secondary hematopoiesis in fetal liver |
| Angiopoietin-1 | E12.5 | Abnormal cardiovascular system |
| bcl-x | E13.0 | Cell death in the peripheral ganglia, and increased cell death of immature hematopoietic cells in fetal liver |
| cas | E11.5-12.5 | Congestion due to systolic dysfunction of vascular smooth muscles, and cardiac hypoplasia |
| EKLF | up to E13.0 | Extreme anemia |
| EPO | E13.0 | Abnormal secondary hematopoiesis in fetal liver |
| EPOR | E13.0 | Abnormal secondary hematopoiesis in fetal liver |
| c-jun | E11.5-15.5 | Increase of nucleated erythrocytes in fetal liver |
| keratin-8 | E12.0-15.5 | Increase of nucleated erythrocytes in fetal liver |
| c-myb | E15.5 | Abnormal secondary hematopoiesis in fetal liver |
| RB | E12.0-16.0 | Abnormal central nervous system, and abnormal hematopoietic system |
| sox4 | E14.0 | Cardiac malformation |
| Tie-1 | E13.5-14.5 | Abnormal maintenance of vascular cells |
| VCAM-1 | E11.5-12.5 | Placental and cardiac malformation |

### Excerpt from Knockout Mice Data Book

To prove the decrease of hematopoietic cells in syno^{-/-} embryos, the present inventors demonstrated an experiment using MEFs derived from syno^{-/-} embryos. Herein, the MEFs exhibited a high and selective susceptibility to ER stress *in vitro*, and apoptotic cell death in syno^{-/-} embryos could be induced by ER stress. This experiment demonstrates that MEFs derived from the syno^{-/-} embryos may become model cells for studying ER stress-induced apoptosis, and can also be utilized as cells to screen for pharmaceutical agents for preventing or inhibiting this apoptosis.

The pharmaceutical formulations of the present invention for hematopoietic disorders, which comprise synoviolin and such as effective ingredients, are expected to be effective as therapeutic agents for patients who have developed a hematopoietic disease due to decreased function or expression of synoviolin caused by genetic factors or secondary environmental factors.

## Claims

1. A method for treating hematopoietic diseases, wherein the method comprises administering any one of the proteins selected from the group consisting of (a) to (d) shown below, or a polynucleotide encoding the protein:
(a) a protein comprising the amino acid sequence of SEQ ID NO: 2;
(b) a protein comprising an amino acid sequence with one or more amino acid substitutions, deletions, insertions, and/or additions in the amino acid sequence of SEQ ID NO: 2, wherein the protein is functionally equivalent to a protein comprising the amino acid sequence of SEQ ID NO: 2;
(c) a protein encoded by a polynucleotide that hybridizes under stringent conditions with a polynucleotide comprising the nucleotide sequence of SEQ ID NO: 1, wherein the protein is functionally equivalent to a protein comprising the amino acid sequence of SEQ ID NO: 2; and
(d) a protein encoded by a polynucleotide comprising a nucleotide sequence with at least 70% or higher homology to the nucleotide sequence of SEQ ID NO: 1, wherein the protein is functionally equivalent to a protein comprising the amino acid sequence of SEQ ID NO: 2.

2. The method of claim 1, wherein the hematopoietic diseases are diseases caused by abnormal erythroblast differentiation.

3. The method of claim 1, which comprises introducing into hematopoietic stem cells a vector harboring the polynucleotide in an expressible state.

4. A method for inducing erythroblast differentiation, wherein the method comprises expressing in hematopoietic stem cells any one of the proteins selected from the group consisting of (a) to (d) shown below:
(a) a protein comprising the amino acid sequence of SEQ ID NO: 2;
(b) a protein comprising an amino acid sequence with one or more amino acid substitutions, deletions, insertions, and/or additions in the amino acid sequence of SEQ ID NO: 2, wherein the protein is functionally equivalent to a protein comprising the amino acid sequence of SEQ ID NO: 2;
(c) a protein encoded by a polynucleotide that hybridizes under stringent conditions with a polynucleotide comprising the nucleotide sequence of SEQ ID NO: 1, wherein the protein is functionally equivalent to a protein comprising the amino acid sequence of SEQ ID NO: 2; and
(d) a protein encoded by a polynucleotide comprising a nucleotide sequence with at least 70% or higher homology to the nucleotide sequence of SEQ ID NO: 1, wherein the protein is functionally equivalent to a protein comprising the amino acid sequence of SEQ ID NO: 2.

5. A pharmaceutical formulation for treating hematopoietic diseases, wherein the formulation comprises as an effective ingredient any one of the proteins selected from the group consisting of (a) to (d) shown below, or a polynucleotide encoding the protein:
(a) a protein comprising the amino acid sequence of SEQ ID NO: 2;
(b) a protein comprising an amino acid sequence with one or more amino acid substitutions, deletions, insertions, and/or additions in the amino acid sequence of SEQ ID NO: 2, wherein the protein is functionally equivalent to a protein comprising the amino acid sequence of SEQ ID NO: 2;
(c) a protein encoded by a polynucleotide that hybridizes under stringent conditions with a polynucleotide comprising the nucleotide sequence of SEQ ID NO: 1, wherein the protein is functionally equivalent to a protein comprising the amino acid sequence of SEQ ID NO: 2; and
(d) a protein encoded by a polynucleotide comprising a nucleotide sequence with at least 70% or higher homology to the nucleotide sequence of SEQ ID NO: 1, wherein the protein is functionally equivalent to a protein comprising the amino acid sequence of SEQ ID NO: 2.

6. The pharmaceutical formulation of claim 5, which is a hematopoietic stem cell harboring the polynucleotide in an expressible state.

7. A method for treating hematopoietic diseases, wherein the method comprises administering an agent that enhances the activity of a protein comprising the amino acid sequence of SEQ ID NO: 2.

8. A therapeutic agent for hematopoietic diseases, which comprises an agent that enhances the activity of a protein comprising the amino acid sequence of SEQ ID NO: 2 as an effective ingredient.

9. A non-human hematopoietic disease model animal embryo whose synoviolin gene function is defective.

10. The non-human hematopoietic disease model animal embryo of claim 9, wherein the non-human animal is a rodent.

11. The non-human hematopoietic disease model animal embryo of claim 10, wherein the rodent is a mouse.

12. The non-human hematopoietic disease model animal embryo of claim 11, which is an embryo of E13.5 or younger.

13. The non-human hematopoietic disease model animal embryo of claim 9, wherein the hematopoietic disease is a disease caused by abnormal erythroblast differentiation.

14. A hematopoietic disease model cell derived from the non-human hematopoietic disease model animal embryo of any one of claims 9 to 13.

15. The hematopoietic disease model cell of claim 14, which is a fibroblast.

16. A method of screening for therapeutic agents for hematopoietic diseases, wherein the method comprises administering a test substance to a non-human hematopoietic disease model animal embryo whose synoviolin gene function is defective, and evaluating the condition of erythroblasts in the non-human animal embryo.

17. A method of screening for therapeutic agents for hematopoietic diseases, wherein the method comprises the steps of:
(a) administering or contacting an ER stress-inducing agent with a non-human hematopoietic disease model animal embryos whose synoviolin gene function is defective, or a cell derived from the embryos;
(b) administering or contacting a test substance to the hematopoietic disease model non-human animal embryos whose synoviolin gene function is defective, or cells derived from the embryos, before, after, or simultaneously with step (a); and
(c) determining cells in which apoptosis was induced from among the non-human animal embryos or the cells derived from the embryos.

18. A method of screening for ER stress-removing agents, wherein the method includes the steps of:
(a) acting an ER stress-inducing agent on cells whose endogenous synoviolin gene function is defective;
(b) contacting a test substance to the cells before, after, or simultaneously with step (a); and
(c) determining cells in which apoptosis was induced from among the cells.
